# EUROPEAN PATENT SPECIFICATION

(11) **EP 0 731 697 B1**
(45) Date of publication and mention of the grant of the patent: **18.04.2001**
(21) Application number: 95904123.7
(22) Date of filing: 23.11.1994
(51) Int. Cl.: A61K 31/165, A61K 31/275

(54) **SSI TYRPHOSTINS AND PHARMACEUTICAL COMPOSITIONS**
SSI TYRPHOSTINE UND PHARMAZEUTISCHE ZUSAMMENSETZUNGEN
TYRPHOSTINES SSI ET COMPOSITIONS PHARMACEUTIQUES

(30) Priority: 24.11.1993 IL 10773693
(43) Date of publication of application: 18.09.1996
(73) Proprietor: YISSUM RESEARCH DEVELOPMENT COMPANY OF THE HEBREW UNIVERSITY OF JERUSALEM, Jerusalem 91042 (IL); Kupot- Holim Health Insurance Institute of the General Federation of Labour in Eretz-Israel, Tel-Aviv (IL); LEVITZKI, Alexander, Patomic, MA 20854 (US)
(72) Inventor: LEVITZKI, Alexander, Patomic, MA 20854 (US); NOVOGRODSKY, Abraham, 76469 Rehovot (IL); GAZIT, Aviv, Jerusalem (IL)
(74) Representative: Viering, Jentschura & Partner
(86) International application number: US9413535
(87) International publication number: WO9514464

(56) References cited:
- EP-A- 0 322 738
- EP-A- 0 444 899
- EP-A- 0 537 742
- WO-A-91/16892
- WO-A-94/26260
- SCIENCE, vol.264, 27 May 1994, USA pages 1319 - 1322 A. NOVOGRODSKY ET AL 'Prevention of Lipopolysaccharide-Induced Lethal Toxicity by Tyrosine Kinase Inhibitors' cited in the application
- DATABASE WPI Week 8713, Derwent Publications Ltd., London, GB; AN 87-089881 & JP,A,62 039 558 (KANEGAFUCHI CHEM KK) 20 February 1987
- JOURNAL OF MEDICINAL CHEMISTRY, vol.32, no.10, 1989, USA pages 2344 - 2352 A. GAZIT ET AL 'Tyrphostins I: Synthesis and Biological Activity of Protein Tyrosine Kinase Inhibitors' cited in the application
- JOURNAL OF MEDICINAL CHEMISTRY, vol.34, no.6, 1991, USA pages 1896 - 1907 A. GAZIT ET AL 'Tyrphostins. 2. Heterocyclic and alpha-Substituted Benzylidenemalononitrile Tyrphostins as Potent Inhibitors of EGF Receptor and ErbB2/neu Tyrosine Kinases' cited in the application
- PROCEEDINGS OF THE NATIONAL ACADEMY OF SCIENCES, USA, vol.88, May 1991, USA pages 4148 - 4152 S. L. WEINSTEIN ET AL 'Bacterial lipopolysaccharide stimulates protein tyrosine phosphorylation in macrophages' cited in the application
- THE FASEB JOURNAL, vol.6, no.14, November 1992, USA pages 3275 - 3282 A. LEVITZKI 'Tyrphostins: tyrosine kinase blockers as novel antiproliferative agents and dissectors of signal transduction' cited in the application
- SCIENCE, vol.242, no.4880, 11 November 1988, USA pages 933 - 935 P. YAISH ET AL 'Blocking of EGF-Dependent Cell Proliferation by EGF Receptor Kinase Inhibitors' cited in the application
- BIOCHEMISTRY JOURNAL, vol.267, no.1, 1990, GB pages 91 - 98 M. KOHNO ET AL 'Mitogenic signalling pathway of tumour necrosis factor involves the rapid tyrosine phosphorylation of 41000-Mr and 43000-Mr cytosol proteins' cited in the application
- PROCEEDINGS OF THE NATIONAL ACADEMY OF SCIENCES, USA, vol.87, May 1990, USA pages 3629 - 3632 R. G. KILBOURN ET AL 'NG-Methyl-L-arginine inhibits tumor necrosis factor-induced hypotension: Implications for the involvement of nitric oxide' cited in the application

## Description

### Field of the Invention

The present invention relates generally to the fields of chemistry, biochemistry and medicine and more specifically to the fields of tyrphostins and inflammatory disorder treatments.

### Background of the Invention

The following description of background art is not admitted to be prior art to the present invention.

Systemic infection with Gram-negative bacteria may result in hypotension and multi-organ dysfunction, a syndrome called septic shock, (Morrison, D.C., and Ryan, J.L., Ann. Rev. Med. 38: 417-432, 1987. The clinical syndrome of Gram-negative septic shock appears to result primarily or exclusively from excessive stimulation of the host immune system, especially macrophages, by the lipopolysaccharide (LPS) or endotoxin which is a complex glycolipid component of the outermost membrane of Gram-negative bacteria, (Guenter et al., 26 J. Appl. Physiol. 780, 1969; Raetz et., FASEB J., 5: 2652-2660, 1991).

LPS is a powerful pleotropic stimulant of immune cells, mainly macrophages that acts by generating cytokines such as TNF-α, IL-I, and IL-6 as well as prostanoids, leukotriens (Beutler and Cerami, 57 A. Ann. Rev. Biochem. 505, 1988) and nitric oxide (Ding et al.; 141 J. Immunol. 2407, 1988; and Zang and Morrison, 177 D.C. J. Exp. Med. 511, 1993). Recent reports demonstrate stimulation of tyrosine phosphorylation of a 41 kDa protein in murine macrophages treated with LPS, (Weinstein, S.L. et al., Proc. Natl. Acad. Sci. USA, 88: 4148-4152, 1991) and the LPS induced phosphorylation of p56 Lck (Corcoran et al., J. Biol-Chem. 268: 20725-20728, 1993).

LPS induction of cytokine release, particularly TNF*-α* and IL-1, is probably the central event in LPS-induced lethal toxicity and in the pathophysiology of Gram-negative bacterial septicemia (Tracey K.J., et al, Science 234: 470-474, 1986). Many of the toxic manifestations of LPS, including adult respiratory distress syndrome and vascular leak syndrome, can be induced by TNFα (tumor necrosis factor-α) and IL-1 beta which synergize with each other (Okusawa et al., 81 J. Clin. Invest. 1162, 1988; and Everaedt et al., 163 Biochem. Biophys. Res. Commun. 378, 1989). Excessive or inappropriate cytokine production is also associated with pathogenic inflammatory conditions such as rheumatoid arthritis, psoriasis and AIDS-related cachexia. The following publications relate to rheumatoid arthritis, psoriasis, and AIDS: Elliott and Maini, 104 Int'l Archives of Allergy and Immun., 112, 1994; Bloxham, 9 Expert Opin. Invest. Drugs, 907, 1994; Bonifati et al., 19 Clin. Exp. Dermatol. 383, 1994; Takematsu et al., 5 J. Dermatol. Treat. 133, 1994; Aukrust et al., 169 J. of Infectious Diseases 420, 1994; Glass et al., 43 Neurology 2230, 1993; and Dezube et al., 6 J. Acquired Immune Deficiency Syndrome 787, 1993.

Nitric oxide, a reactive nitrogen intermediate has been implicated in mediation of some of the anti-tumor and parasite killing effect of macrophages (Stuehr, D.J., and Marietta, M.A., J. Exp. Med., 169: 1543-1555, 1989). Some of the toxic manifestations of LPS may be mediated by NO. (Kilbourn, R.G., Proc. Natl. Acad. Sci. USA, 87, 3629-3632, 1990). LPS, by itself and in combination with IFN-γ was shown to stimulate nitric oxide in mouse peritoneal macrophages, (Ding. A.H., J. Immunol., 141: 2407-2412, 1988. Zhang, X. and Morrison, D.C. J. Immunol., 150: 1011-1018, 1993). Production of NO is induced by LPS and inhibited by some tyrphostins (Tsunawaki and Nathan, 259 J. Biol. Chem 4305, 1984).

Tyrphostins are specific inhibitors of protein tyrosine kinases and were designed to interfere with the substrate binding site of tyrosine kinases. Thus, tyr-phostins exhibit selectivity in their ability to inhibit different protein tyrosine kinases and distinct biological responses. Tyrphostins are described in Allen et al., Clin. Exp. Immunol. 91:141-156 (1993); Anafi et al., Blood 82:12:3524-3529 (1993); Baker et al., J. Cell Sci. 102:543-555 (1992); Bilder et al., Amer. Physiol. Soc. pp. 6363-6143:C721-C730 (1991); Brunton et al., Proceedings of Amer. Assoc. Cancer Rsch. 33:558 (1992); Bryckaert et al., Experimental Cell Research 199:255-261 (1992); Dong et al., J. Leukocyte Biology 53:53-60 (1993); Dong et al., J. Immunol. 151(5):2717-2724 (1993); Gazit et al., J. Med. Chem. 32:2344-2352 (1989); Gazit et al., J. Med. Chem. 36:3556-3564 (1993); Kaur et al., Anti-Cancer Drugs 5:213-222 (1994); Kaur et al., King et al., Biochem. J. 275:413-418 (1991); Kuo et al., Cancer Letters 74:197-202 (1993); Levitzki, A., The FASEB J. 6:3275-3282 (1992); Lyall et al., J. Biol. Chem. 264:14503-14509 (1989); Peterson et al., The Prostate 22:335-345 (1993); Pillemer et al., Int. J. Cancer 50:80-85 (1992); Posner et al., Molecular Pharmacology 45:673-683 (1993); Rendu et al., Biol. Pharmacology 44(5):831-838 (1992); Sauro and Thomas, Life Sciences 53:371-376 (1993); Sauro and Thomas, J. Pharm. and Experimental Therapeutics 267(3):119-1125 (1993); Wolbring et al., J. Biol. Chem. 269(36):22470-22472 (1994); U.S. Patent No. 5,217,999; and Yoneda et al., Cancer Research 51:4430-4435 (1991);

LPS induces protein tyrosine phosphorylation (Weinscein et al., 88, Proc. Natl. Acad. Sci. U.S.A. 4148, 1391) in macrophages as well as the generation of eicosanoids (Glaser et al., 45 Biochem, Pharmacol. 711, 1993), and some tyrphostins and herbimycin A inhibit these events (Weinstein et al., supra.; Glaser et al., supra.). LPS induces in macrophages the ability to kill tumor cells and these tumoricidal properties can be blocked by some tyrphostins (Dong et al., 53 J. Leukocyte Biol. 53, 1993). TNF-α (Kohn et al., 267 Biochem. J., 91, 1990; Evans et al., 75 Blood 88, 1990; and Vietor et al., 268 J. Biol. Chem. 18994, 1993) and IL-1β (Munoz et al., 22 Eur. J. Immunol. 1391, 1992; and Guy et al., 266 J. Biol. Chem. 14343, 1991) also induce tyrosine phosphorylation in target cells and the signaling events induced by these ligands are blocked by PTK inhibitors such as certain tyrphostins (Yaish et al., Science, 1988; and Levitzki 6 FASEB J. 3275, 1992), herbimycin A (Dong et al., supra.; and Iwasaki et al., 298 FEBS letters 240, 1992) and genistein (Glaser et al., supra.; and Coyne and Morrison, 173 Biochem, Biophys, Res. Commun. 718, 1990).

### Summary of the Invention

The present invention relates to products useful for the prevention and/or treatment of various disorders, in particular inflammatory disorders such as septic shock, rheumatoid arthritis, psoriasis and complications of HIV infection. These disorders involve an excessive stimulation of the immune system by various agents (for example, LPS) which may lead to production of TNF-α and other cytokines which play a major role in a variety of disorders. Featured are novel compounds and pharmaceutical compositions, both of which may be used for prevention and/or treatment as described herein, as well as methods for making the novel compounds. The invention provided is thus useful for the prevention of, or for the alleviation of symptoms of inflammatory disorders. The active ingredients of the novel compositions are certain tyrphostin compounds, some of which are novel and some of which have been described before.

A variety of tyrphostins from different families were tested in assays that measure different aspects of pathological inflammatory response. Administration of tyrphostins significantly reduces lethal-toxicity induced by LPS in mice, both when given prior to LPS and up to 2 hours after LPS. The tyrphostins tested are shown in Table 1 and in the Examples below. The protection against LPS induced toxicity correlates with the ability of these agents to block production of tumor necrosis factor alpha (TNFα) and nitric oxide in macrophages as well as production of TNFα in vivo. The inhibitory effect correlates with the potency of the tyrphostins to block tyrosine phosphorylation of a p42^{MAPK} protein substrate in the murine macrophage.

Certain tyrphostins have been shown to inhibit a limited class of in vitro activities such as nitric oxide production and tyrosine phosphorylation. Applicant has now shown the in vivo effectiveness of tyrphostins in preventing LPS induced toxicity, reducing LPS induced increases in TNFα levels, and preventing TNFα induced toxicity and has identified the particular class of tyrphostins that possess the above mentioned in vitro and/or in vivo activities and has identified the novel tyrphostins described herein.

Many acute and chronic pathogenic inflammatory conditions have been associated with excessive or inappropriate cytokine production, in particular TNF-α. A number of therapeutic substances have been tested in humans in hopes of reducing the symptoms associated with inappropriate cytokine response. The tyrphostins of the present invention may be superior to, for example, anti-TNF monoclonal antibodies as they may be administered orally and are unlikely to stimulate an unwanted anti-therapeutic immune response such as HAMA. In addition, the tyrphostins of the present invention are catalytic inhibitors which may allow them to be active at far lower doses than biologics whose mode of action is to essentially act as a sponge to bind-up and clear excess cytokines.

Thus, in a first aspect, the present invention relates to a pharmaceutical composition which contains a physiologically acceptable carrier or diluent and a therapeutically effective amount of a SSI tyrphostin compound as defined in claim 4.

By "physiologically acceptable carrier or diluent" it is meant a non-toxic substance and is a phrase that is well-known to those skilled in the art. Several examples of physiologically acceptable carriers or diluents are described herein. In preferred embodiments the carrier or diluent is a material that is not commonly used to buffer the pH of a solution, such as Tris buffer.

By "therapeutically effective amount" it is meant agents of this invention have a "therapeutic effect" which generally refers to either the reduction in symptoms associated with inflammatory disorders such as organ dysfunction, painful swelling of tissues, cachexia, shock, hypotension, etc., or the inhibition, to some extent, of the production of causes or contributors to such a disorder, for example nitric oxide production, excessive tyrosine phosphorylation, or cytokine (e.g., TNFα) production. In particular, the therapeutic effect includes the prevention or delay of death or organ failure. The doses of SSI tyrphostins which are useful as a treatment are "therapeutically effective" amounts. Thus, as used herein, a "therapeutically effective amount" means an amount of the SSI tyrphostin which produces the desired therapeutic effect. This amount can be routinely determined by one of skill in the art and will vary depending upon several factors such as the particular illness from which the patient suffers and the severity thereof, as well as the patient's height, weight, sex, age, and medical history. Generally, SSI tyrphostins of the present invention are preferably provided at a dose of between 1 mg/kg to 50 mg/kg. More specifically, one preferable dose range is from 10 to 40 mg/kg and another is between. 20 and 30 mg/kg.

By "SSI tyrphostin" is meant a compound of the general formula 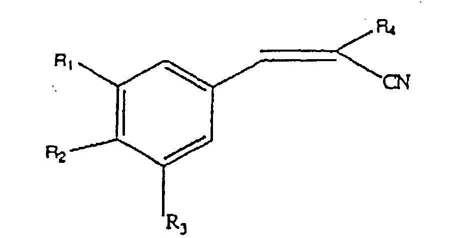 wherein
R₁, designates -OH, -NO₂-, lower alkoxy, or C-(CH₃)₃;
R₂ designates -OH, or -NO₂-;
R₃ designates -H, NO₂- halogen or -C-(CH₃)₃; and
R₄ designates -CN, -COOH, 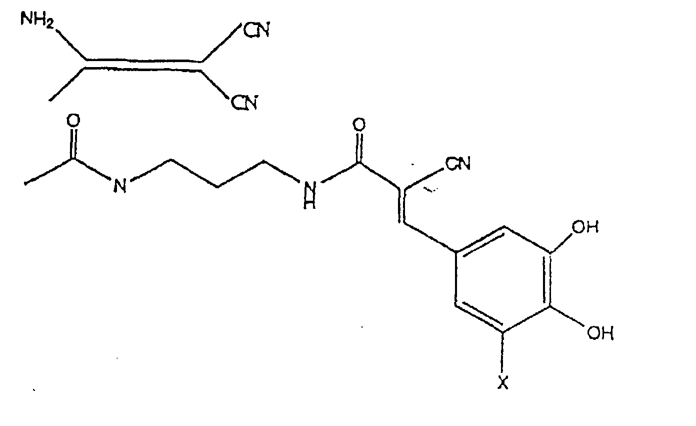 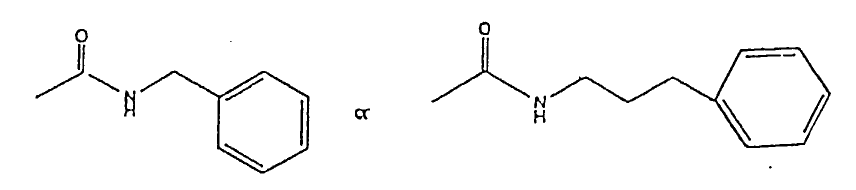
and X designates -H or nitro.

By "alkoxy" is meant an "-O-alkyl" group, where "alkyl" refers to a saturated aliphatic hydrocarbon, including straight-chain, branched-chain, and cyclic alkyl groups. Preferably, the alkyl group has 1 to 12 carbons. More preferably it is a lower alkyl of from 1 to 7 carbons, more preferably 1 to 4 carbons. The alkyl group may be substituted or unsubstituted. When substituted the substituted group(s) is preferably, hydroxyl, cyano, alkoxy, =O, =S, NO₂-, N(CH₃)₂, amino, or SH.

Preferred are compounds SSI 3 (where R₁ is hydroxy, R₂ is nitro, R₃ is hydrogen and R₄ is cyano), or SSI 12 (where R₁ is methoxy, R₂ is hydroxy, R₃ is nitro and R, is a carboxyl group), or SSI 6 (where R is nitro, R₂ is hydroxy, R₃ is hydrogen and R₄ is carboxyl). Preferebly, the compositions are adapted for administration by injection or by the oral route. Generally the unit dosage form contains from 1 mg/kg to 50 mg/kg (also preferable is the range from 10 mg/kg to 40 mg/kg, and the range from 20 mg/kg to 30 mg/kg) of the active compound, but this can vary according to the route of administration and the exact nature of the compound. It is preferred to administer the compositions at an early stage in order to give a maximum degree of prevention of, or alleviation of the effect of bacteria inducing septic shock.

In another aspect the invention features the use of a SSI tyrphostin as defined in claim 6 for preparing a pharmaceutical composition for treating an inflammatory disorder.

Preferred compounds, dosages, and routes of administration are as above. Preferred organisms to be treated include mammals, in particular mice, rabbits, dogs, cats, sheep, monkeys and humans. Those skilled in the art are familiar with various animal models that may be used to further test the tyrphostins identified herein as being lead candidates for the treatment of various disorders in humans. Preferred disorders include inflammatory disorders, especially those selected from the group consisting of septic shock, rheumatoid arthritis, psoriasis and conditions associated with AIDS such as cachexia and HIV-1, chronic granulomutotic diseases, tuberculosis, leprosy, meurological inflammatory conditions, multiple sclerosis, graft versus host disease and atherosclerosis.

In another aspect the present invention provides a novel SSI tyrphostin compound selected from the group consisting of SSI 6, SSI9, SSI 19, SSI 20, SSI 21, SSI 22, SSI 23, and SSI 24

In another aspect the invention features a method of making a SSI tyrphostin compound selected from the group consisting of SSI 19, SSI 20, SSI 21, SSI 22, SSI 23, and SSI 24 comprising the steps of exposing a benzaldehyde or substituted benzaldehyde compound to a tyrphostin or malono nitrile corresponding to a final tyrphostin of said group.

In another aspect the invention features the use of a SSI tyrphostin for preparing a pharmaceutical composition for preventing LPS induced toxicity. By "LPS induced toxicity" is meant for example, death caused by an abnormal or elevated level of LPS. An abnormal or elevated is level is one recognized by those skilled in the arc as being statistically different from a normal individual. In preferred embodiments the SSI tyrphostin is selected from the group consisting of SSI 3, SSI 4, SSI 6, SSI 12, SSI 16, SSI 17, and SSI 23. In another aspect the invention features the use of a SSI tyrphostin for preparing a pharmaceutical composition for reducing an LPS induced increase in TNF-α levels.

By "LPS induced increase in TNF-α levels" is meant that the amount of TNF-α in an organism is increased by the presence of LPS. Bioassays and ELISA techniques, for example, may be used to measure TNF-α levels. In preferred embodiments the SSI tyrphostin is selected from the group consisting of SSI 2, SSI 3, SSI 6, SSI 9, SSI 10, SSI 11, SSI 12, SSI 17 and SSI 23.

In another aspect the invention features the use of a SSI tyrphostin for preparing a pharmaceutical composition for preventing TNF-α induced toxicity. By "TNFα induced toxicity" is meant death caused an abnormal or elevated level of TNFα. In preferred embodiments the SSI tyrphostin is selected from the group consisting of SSI 3, SSI 16, SSI 17, SSI 18, SSI 19, and SSI 23.

In another aspect the invention features the use -of a SSI tyrphostin for preparing a pharmaceutical composition for inhibiting production of NO₂⁻.

In preferred embodiments the tyrphostin is selected from the group consisting of SSI 3, SSI 6, SSI 8, SSI 9, SSI 10, SSI 11, SSI 16, and SSI 17.

In another aspect the invention features the use of a SSI tyrphostin for preparing a pharmaceutical composition for treating inflammation characterized by TNF-α related activity.

In preferred embodiments, the disorder is sepsis, psorasis, or AIDS related cachexia.

Other features and advantages of the invention will be apparent from the following description of the preferred embodiments thereof, and from the claims.

### Description of the Preferred Embodiments

The present invention relates to the prevention and/or treatment of various inflammatory disorders, in particular septic shock, that result from excessive stimulation of the immune system by an agent such as LPS, and various disorders where excessive levels of cytokines including TNF play a major role. Protein tyrosine kinase inhibitors of the tyrphostin family protect mice against LPS induced lethal toxicity and the protection correlates with the ability of these agents to block production of tumor necrosis factor alpha (TNFα) and nitric oxide in macrophages as well as production of TNFα in vivo.

Pretreatment of mice with tyrphostins, specific inhibitors of protein tyrosine kinases, markedly reduced lethal-toxicity induced by LPS (Tables 5 and 6). Certain tyrphostins also prevented lethal toxicity in mice even when administered 2 hrs following administration of LPS. We have also demonstrated that certain tyrphostins inhibit LPS induced increase of serum TNF-α levels in mice. In addition we have found that certain tyrphostins reduce TNF-α induced lethal toxicity in mice (Table 8) and also inhibit TNF-α induced cytotoxicity against susceptible cells in vitro (Table 4).

Tyrphostins, which belong to different families, were screened for their ability to inhibit LPS-induced production of TNF-α by activated murine peritoneal macrophages *in vitro* (Table 1). Among the tyrphostins tested, SSI 3 and SSI 8, were the most potent. These tyrphostins have no effect on EGF receptor, Her-2/neu receptor or PDGFR even at concentrations above 100 uM (Gazit et al., 32 J. Med. Chem. 2344, 1989). These tyrphostins also inhibit the *in vitro* production of NO₂- (an oxidative product of nitric oxide). SSI 3 and SSI 8, but not SSI 6 and SSI 9 (Table 1), were also active in blocking TNF-α induced cytotoxicity *in vitro* (Table 2). SSI 3 was most effective in preventing LPS-induced lethal toxicity when administered prior to LPS injections, such as 2 hours before LPS injections. Administration of tyrphostin SSI 3 2 hr after LPS had essentially no protective effect. In contrast, SSI 17 was effective when administered 2 hrs after LPS.

There are multiple biological responses to LPS that are relevant to the pathogenesis of LPS toxicity and Gram-negative sepsis. They include effects on monocytes/ macrophages, neutrophils, endothelial cells, B-cells, epithelial cells, platelets and complement. Most of these responses result from inductive processes that are associated with membrane signal transduction. Protein tyrosine phosphorylation was enhanced upon stimulation of macrophages with LPS and of fibroblasts with TNF-α. Thus, inhibitors of tyrosine kinases such as tyrphostins can protect against toxicity induced by these agents.

As shown, tyrphostins exert a dramatic protective effect against LPS-induced lethal toxicity. Of the effects studied, only LPS-induced accumulation of granulocytes in the lungs and alteration in blood lymphocyte and granulocytes were not affected by SSI 3. Tyrphostins were most effective when administered prior to challenge with a high dose of LPS (1.5 mg/mouse) and some were also effective after LPS administration. The indication is therefore that tyrphostins are effective in preventing septic shock following Gram-negative sepsis, experimentally or clinically.

Treatment of mice with the tyrphostin SSI 3 reduced lethal toxicity induced by LPS. The protective effect of SSI 3 correlates with its inhibition of TNF-α production, NO production, and protein phosphorylation. We have employed a model in which LPS doses of LD₉₅ were employed. Under these conditions SSI 3 conferred nearly full protection when injected prior to LPS and reduced protection when administrated later. The pathophysiological process as it takes place in humans actually involves the gradual release of LPS by the infecting Gram negative bacteria. The experimental protocol as described is much more dramatic since lethal doses of LPS are administered in a single dose. It is therefore anticipated that tyrphostins may be effective in preventing septic shock when administered at the onset of the clinical signs of sepsis or septic shock.

Tyrphostins prevent the onset of LPS toxicity as well as the action of LPS induced cytokines, thus, PTK inhibitors such as SSI 3 may be effective in preventing the effects of septic shock in Gram negative infections. Other agents, such as steroids (Remick et al., 60 Laborat. Invest. 766, 1989) or chlorpromazine (Gadina et al., 173 J. Exp. Med. 1305, 1991) prevent LPS toxicity by mechanisms that are distinct from that mediated by tyrphostins. These agents were also shown to be effective by applying them prior to lethal doses of LPS. It is possible that treatment of septic shock by a combination of these agents may be more effective than by each agent alone.

Tumor Necrosis Factor α (TNF-α) has also been reported to play a key role in the pathogenesis of chronic inflammatory diseases, such as rheumatoid arthritis and atherosclerosis. It is clear that the compounds of the present invention are also effective in alleviating the symptoms of these diseases. This can be deduced from experiments with laboratory animals with models of such diseases, such as in adjuvant induced arthritis and in the atherosclerosis model in WHHA Rabbit. Because the tyrphostins reported on inhibit both TNF-α production and its action induced by LPS it is extremely feasible that other pathophysiological conditions associated with TNF-α can also be managed by these compounds. These include: HIV-1 infection and chronic granulomutotic diseases such as tuberculosis, leprosy, neurological inflammatory conditions (such as multiple sclerosis) and GVH (graft versus host diseases).

### I. SSI Tyrphostins Prevent LPS Induced Lethal Toxicity In Vivo

Surprisingly, SSI 3 was shown to markedly prevent lethal toxicity induced by LPS, for example when administered (injected i.p.) 2 hours prior to LPS. LPS, at a dose of 1.5 mg/mouse induced 95% lethality within 5 days (19 mice out of 20). Administration of SSI 3 (400 ug/ mouse), two hrs prior to LPS, reduced the extent of lethality to 10% (2 mice out of 20). A PBS/DMSO control was used.

The animals were sick in both experimental groups during the first 36 hours, were immobile and had diarrhea. Thereafter, the animals who had been treated with SSI 3 gradually recovered and on the 5th day appeared normal. There were no visible toxic manifestations in mice that were treated with SSI 3 alone. Thereafter, most of the animals that had been treated with SSI 3 gradually recovered, and on the fifth days they appeared normal. There were no visible toxic manifestations in mice that were treated with SSI 3 alone. The animals of both groups were followed for additional 3 weeks; no life shortening or any toxic effects were noted. Administration of SSI 3 of up to 12 mg per mouse (30 times the 400 ug per mouse given in these experiments), did not show any toxicity as revealed by appearance in the treated animals, hematological findings and macroscopic pathological analysis.

The protective effect of SSI 3 against LPS-induced toxicity was dose dependent. SSI 3 at 400 ug/mouse was the minimal dose that provided essentially full protection against LPS (1.5 mg/mouse) induced lethal toxicity when administered 2 hours prior to LPS; although doses of 100 and 200 ug/mouse provided partial protection in studies with 5 mice over 6 days. In contrast, SSI 17 was effective when administered 2 hrs after LPS. The effect of the timing of SSI 3 administration in relationships to the time of LPS treatment was also investigated. Administration of SSI 3 at the time of LPS treatment was less effective in preventing the lethal toxicity than when administered 2 hr prior to LPS. Administration of SSI 3 2 hours after LPS had essentially no protective effect on the LPS-induced lethal toxicity in studies with 5 mice over 6 days. In contrast, SSI 17 was effective when administered 2 hrs after LPS.

In addition to SSI 3, other tyrphostins at 200 ug/ mouse (SSI 4, SSI 12, and SSI 6) were active, at different degrees (SSI 6 was best - 5 out of 5 mice survived, followed by SSI 4 - 4 out of 5 mice , SSI 3 - 3 out of 5 mice, and then SSI 12 - 2 out of 5 mice), in preventing lethal toxicity induced by LPS (1.5 mg/mouse). The dosing of the protective effect of SSI 3 correlates with its inhibition of TNF-α production *in vivo.*

### II. SSI Tyrphostin Prevents LPS Induced Increases In Serum TNFα Levels

TNF-α was implicated to mediate many of the toxic effect of LPS. For example, the effect of SSI 3 on serum TNF-α levels in mice which had been treated with LPS was investigated. LPS induced a rapid increase in serum TNF-α levels. Administration (i.p. injection) of SSI 3 at 400 ug/mouse (C56BL mice 6 to 8 weeks old) 2 hrs prior to LPS treatment markedly prevented the increase in TNF-α (levels in LPS-treated mice levels of 3 or 4 ng/ml versus 7 or 14 ng/ml). (Tables 2 and 7) We used a bioassay and an ELISA for TNF-α determination after 2 hours when the mice were bled by orbital puncture. The bioassay gave somewhat higher levels compared to the ELISA. This finding may indicate that the serum from LPS-treated mice contained in addition to TNF-α, other cytotoxic factors.

### III. SSI Tyrphostin Delays TNFα Induced Toxicity In Vivo

The effect of SSI 3 on lethal toxicity in mice induced by TNF-α was investigated. Mice are relatively resistant to TNF-α, when applied as a single agent. Pretreatment of mice with actinomycin D renders them extremely sensitive to TNF. (Wallach et al., 140 J. Immun. 2994, 1988). SSI 3 delayed TNF-α induced lethal toxicity in actinomycin D treated mice by approximately 15 hours at two ratios of actinimycin D:TNF (20 to 2.5 and 15 to 1.0). Mice were injected, i.p., with TNF at 0 time. SSI 3, 400 mg/mouse was administered (i.p.) 2 hr, and actinomycin D (ACT.D) was given (i.p.) 30 min prior to TNF injection. Each experimental group contained 5 mice. Injection of actinomycin D alone causes death in 2 out of 5 animals after 120 hours and 3 out of 5 after 144 hours.

Tyrphostins also reduced TNF-α induced cytotoxicity, in vitro for tyrphostins SSI 2, 3, 6, and 12. Murine fibroblastic cells (A9) were incubated for 24 hours with TNF at different concentrations in the presence of cyclohexamide (50 ug/ml). Tyrphostins at different concentration were added 2 hr prior to TNF. After 24 hrs cells viability was determined by vital staining using neutral red. Deviation from the mean did not exceed 8%. Two additional experiments yielded similar results.

TNF-α by itself is not effective in inducing lethality in mice. LPS induced lethality involves the synergistic effect of multiple effector molecules such as TNF-α, IL-1, interferon and NO. Therefore, by preventing LPS action one obtains more dramatic results. It is likely however, that a different class of tyrphostins may prove to be more effective in blocking TNF-α toxicity than LPS toxicity. The tyrphostins SSI 16 and SSI 17 were found to be more active in inhibiting TNF-α cytotoxicity *in vitro* by two to three fold. This finding suggests that different sets of PTKs mediate the effects of LPS and TNF-α and therefore different families of tyrphostins will be effective against these two agents.

### IV. SSI Tyrphostins Inhibit NO₂- Production

The tyrphostins SSI 3 and SSI 8 effectively inhibited NO₂- production in unstimulated and LPS-stimulated periodate-activated murine macrophages. Periodate-activated murine peritoneal macrophages were incubated in the absence and presence of LPS (10 mg/ml) and SSI 3 (20 mM and 5 mM). Nitrite (NO₂-) levels were determined in supernatants at the time indicated, as described herein. (Table 5) Values are expressed as means of triplicate cultures. Deviation from the mean did not exceed 5%. Two additional experiments yielded similar results. Under the same experimental conditions SSI 8 had a similar effect.

### V. SSI Tyrphostins Inhibit Tyrosine Phosphorylation

LPS-induced tyrosine phosphorylation of a 42 kD protein in murine peritoneal macrophages was inhibited by pre-treating the cells as before with protective concentrations of SSI 3. This protein band was identified as p42^{MAPK}. The identity of the PTK(s) responsible for the tyrosine phosphorylation of specific macrophage proteins is still unknown, although recent studies however, suggest that LPS binds to CD14 and induces activation of CD14-associated protein tyrosine kinase p53/56^{Lyn} and also of p58/64^{Hck}. (Stefanova et al., supra.)

### VI. Administration

Compounds of the present invention can be administered to a mammalian host in a variety of forms adapted to the chosen route of administration, i.e., orally, or parenterally. Parenteral administration in this respect includes administration by the following routes: intravenous, intramuscular, subcutaneous, intraocular, intrasynovial, transepithelial including transdermal, ophthalmic, sublingual and buccal; topically including ophthalmic, dermal, ocular, rectal and nasal inhalation via insufflation and aerosol and rectal systemic.

The active compound may be orally administered, for example, with an inert diluent or with an assimilable edible carrier, or it may be enclosed in hard or soft shell gelatin capsules, or it may be compressed into tablets, or it may be incorporated directly with the food of the diet. For oral therapeutic administration, the active compound may be incorporated with excipient and used in the form of ingestible tablets, buccal tablets, troches, capsules, elixirs, suspensions, syrups, wafers, and the like. Such compositions and preparations should contain at least 0.1% of active compound. The percentage of the compositions and preparations may, of course, be varied and may conveniently be between about 2 to about 6% of the weight of the unit. The amount active compound in such therapeutically useful compositions such that a suitable dosage will be obtained. Preferred compositions or preparations according to the present invention are prepared so that an oral dosage unit form contains between 1 and 1000 mg of active compound.

The tablets, troches, pills, capsules and the like may also contain the following: A binder such as gum tragacanth, acacia, corn starch or gelatin; excipients such as dicalcium phosphate; a disintegrating agent such as corn starch, potato starch, alginic acid and the like; a lubricant such as magnesium stearate; and a sweetening agent such as sucrose, lactose or saccharin may be added or a flavoring agent such as peppermint, oil of wintergreen, or cherry flavoring. When the dosage unit form is a capsule, it may contain, in addition to materials of the above type, a liquid carrier. Various other materials may be present as coatings or to otherwise modify time physical form, of the dosage unit. For instance, tablets, pills, or capsules may be coated with shellac, sugar or both. A syrup or elixir may contain the active compound, sucrose as a sweetening agent, methyl and propylparabens as preservatives, a dye and flavoring such as cherry or orange flavor. Of course, any material used in preparing any dosage unit form should be pharmaceutically pure and substantially non-toxic in the amounts employed. In addition, the active sustained-release preparations and formulations.

The active compound may also be administered parenterally or intraperitoneally. Solutions of the active compound as a free base or pharmacologically acceptable salt can be prepared in water suitably mixed with a surfactant such as hydroxypropylcellulose. Dispersion can also be prepared in glycerol, liquid polyethylene glycols, and mixtures thereof and in oils. Under ordinary conditions of storage and use, these preparations contain a preservative to prevent the growth of microorganisms.

The pharmaceutical forms suitable for injectable use include sterile aqueous solutions or dispersions and sterile powders for the extemporaneous preparation of sterile injectable solutions or dispersions. In all cases the form must be sterile and must be fluid to the extent that easy syringability exists. It may be stable under the conditions of manufacture and storage and must be preserved against the contaminating action of microorganisms such as bacteria and fungi. The carrier can be a solvent or dispersion medium containing, for example, water, ethanol, polyol (for example, glycerol, propylene glycol, and liquid polyethylene glycol, and the like), suitable mixtures thereof, and vegetable oils. The proper fluidity call be maintained, for example, by the use of a coating such as lecithin, by the maintenance of the required particle size in the case of dispersion and by the use of surfactants. The prevention of the action of microorganisms can be brought about by various antibacterial and antifungal agents, for example, parabens, chlorobutanol, phenol, sorbic acid, thimerosal, and the like. In many cases, it will be preferable to include isotonic agents, for example, sugars or sodium chloride. Prolonged absorption of the injectable compositions can be brought about by use of agents delaying absorption, for example, aluminum monostearate and gelatin.

Sterile injectable solutions are prepared by incorporating the active compound in the required amount in the appropriate solvent with various of the other ingredients enumerated above, as required, followed by filtered sterilization. Generally, dispersions are prepared incorporating the various sterilized active ingredient into a sterile vehicle which contains the basic dispersion medium and the required other ingredients from those enumerated above. In the case of sterile powders for the preparation of sterile injectable solutions, the preferred methods of preparation are vacuum drying and the freeze drying technique which yield a powder of the active ingredient plus any additional desired ingredient from previously sterile-filtered solution thereof.

The therapeutic compounds of this invention may be administered to a mammal alone or in combination with pharmaceutically acceptable carriers, as noted above, the proportion of which is determined by the solubility and chemical nature of the compound, chosen route of administration and standard pharmaceutical practice. The dosage of the present therapeutic agents which will be most suitable for prophylaxis or treatment will vary with the form of administration, the particular compound chosen and the physiological characteristics of the particular patient under treatment. Generally, small dosage will be used initially and if necessary, will be increased by small increments until the optimum effect under the circumstance is reached. The therapeutic human dosage, based on physiological studies using rats, will generally be from 1 mg to 50 mg/kg of body weight per day preferably from 3 to 15 mg per day, although it may be administered in several different dosage units from once to several times a day. Oral administration generally requires higher dosages.

### Examples

The following examples are provided merely to illustrate various preferred embodiments of the present invention and are not meant to limit the scope of the invention as defined in the claims. The following in vitro and in vivo examples demonstrate the tyrphostin activity in preventing LPS induced toxicity, reducing TNF-α serum levels, NO₂- production

SSI 17 and SSI 23 prevent induced lethality even when administered late after LPS. SSI 23 is very soluble in aqueous solution and rapidly precipitates after dilution of the stock (made in DMSO) in PBS. Trials to inject SSI 23 dissolved in a variety of solvents which include alcohol and detergents were discontinued since the solvents themselves affected LPS toxicity. : Injection of SSI 23 will not be a problem in clinical trials due to the extensive dilution of the solvent after administration.

### Material and Methods

### 1. Materials

Stock solutions (50 mM) were made in DMSO. Dilutions were made in PBS. Lipopolysaccharide from E. coli, sero-type 055:B5 prepared using phenol extraction was obtained from Sigma Chemical Company. Recombinant human tumor necrosis factor-α (TNF-α) (5x10 units/mg) was obtained from Reprotech, Inc., Rocky Hill, N.J. Female C58BL mice (6-8 weeks old) were used. The mice were bred in the animal breeding facilities at the Beilinson Medical Center.

### 2. TNF-α determination

The amount of TNF-α was quantitated by assessing the extent of killing of the TNF-α sensitive cell line (A9) essentially as described by Ruff and Gilford, G. E. J. Immunol. 125, 1671-1677, (1980). Briefly, mouse A9 fibroblasts were plated in 96-well flat-bottom micro-titer plates at 30,000 cells /0.1 ml to establish a dense monolayer. After incubation for 24 hr at 37 C in a humidified 5% CO atmosphere, cycloheximide was added to a final concentration of 50 ug/ml and 100 ul of serially diluted test samples were added to the wells. After incubation for additional 18 hr the supernatants were carefully aspirated, the monolayer were washed twice with PBS and 200 ul of neutral red solution (0.02%) was added. After incubation for 2 hr, cells were washed and the dye that had been absorbed by the live cells was extracted using 200 ul of 50% ethanol. The concentration of the dye was determined by an ELISA autoreader using a 550 nm filter. Murine TNF-α ELISA kit from ENDOGEN Inc., was used for quancitation of murine TNF-α.

### 3. NO₂- determination

NO₂- the product of NO oxidation is used to determine NO produced. Nitrite concentration in supernatants of macrophages was measured by a microplate assay method. 100 ul aliquots of supernatants were incubated with an equal volume of Griess reagent (1% sulfanilamide/O.1% naphthylethylene diamine dihydrochloride/2.5% H₃PO₄) at room temperature for 10 min. The absorbance at 550nm was determined in a microplate reader. Sodium nitrite was used as a standard.

### 4. Macrophage culture

Mice were injected i.p. with 1 ml sodium periodate (5mM). Three to 4 days later, macrophages were washed from the peritoneal cavity with PBS. After centrifugation at 170g for 10 min at 40° C, the cell pellet was resuspended in RPMI 1640 containing 10% heat-inactivated new born calf serum. Adherent macrophage monolayers were obtained by plating the cells in 96-well plastic trays at 4x10⁵ cells/well for 2 hr at 37°C in 5% CO₂/air. Nonadherent cells were removed by suction and complete medium was added.

### 5. Mouse blood leukocyte count

Mouse blood leukocyte count and differential analysis were done using Cell-Dyn 1600, Hematology Analyzer (Sequoia Turner Corporation, U.S.A).

### 6. Tyrphostin synthesis

Tyrphostins were synthesized according to the methodologies previously described in U.S. Patent Application No. 08/236,420, filed April 28, 1994, Gazit, A., et al., J. Med. Chem. 32: 2344-2352, (1989) and Gazit, A., et al., J. Med. Chem. 34: 1897-1907, (1991). The synthesis of SSI 1, SSI 2, SSI 3, SSI 4 and SSI 5 was described previously: Gazit et Med. Chem., 32, 2344 (1989). The other tyrphostins were prepared by the methods described in the above publication and in: Gazit et al., J. Med. Chem. 34, 1897 (1991) and Novogrodsky et al., Science, 264: 1318 - 1322, (1994).

SSI 6-light-yellow solid, mp-193, 88% yield, NMR acetone d₆ δ 8.92(1H, d,J=2.4Hz,H₂), 8.43(1H,dd,J=8.8, 2.4H_{z},H₆), 8.39 (1H,S,vinyl), 7.43(1H,d, J=8.9H_{z},H₅).

SSI 7-light-yellow solid, mp-218, 76%, yield, NMR acetone d₆ δ 8.42(1H,S, Vinyl), 8.33 (1H,D,J=8.0H_{z},H₅), 7.91(1H, d, J=2.0H_{z}, H₂), 7.76(1H, dd, J=8.0, 2.0, H₂, H₆).

SSI 8-red solid, mp-185, 88% yield. NMR acetone d₆ δ 8.22 (1H, d, J=2.1 H_{z}), 8.15(1H, S, vinyl), 7.86(1H, d,J=2.1H_{z}). MS-232(M+1, 12%), 232(M⁺, 100), 185(17), 183(55), m/e.

SSI 9-yellow solid, mp-230, 73% yield. NMR acetone d₆ δ 8.34, 8.02 (2H, 2d, J=2.0 H_{z}), 8.27 (1H, S, vinyl). MS-251(M+1, 12%), 250(M⁺, 100%), 202(M-NO₂-H₂, 27), 174 (17), 130(18) m/e.

SSI 10-orange solid, mp-163, 30% yield. NMR acetone d₆ δ 8.16, 7.85 (2H, 2d, J=2.0 H_{z}), 8.10(1H, S, vinyl), 7.20(5H,m,Ph), 3.43 (2H,t, J=6.0H_{z}) 2-71(2H, t, J=6.OH_{z}), 1.95(2R,m). MS-202 (H-NO₂-CH₂^{Ph}, 40%), 118(100%), 117 (95), 91 (70), m/e.

SSI 11-red solid, mp-237, 92% yield. NMR acetone d₆ δ 8.16, 7.84(4H,2d,J=2.0 H_{z}), 8.11(2H,S,vinyl), 3.50-3.0(4H,m), 1.8(2H,m).

### Example 1: Synthesis of SSI 19

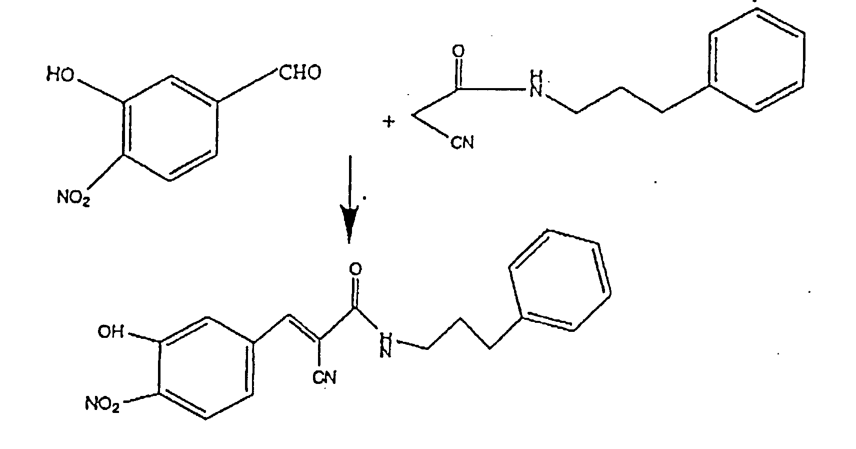

0.35g, 2mM, 3-hydroxy 4-nitro benzaldehyde, 0.42g, 2 mM, Compound 1 (above, top right) and 30 mg β-alanine in 15 ml ethanol were refluxed 4 hours. Evaporation and chromatography gave 100 mg, 14% yield, yellow solid, mp-106. NMR acetone d₆ δ 8.27(1H,d,J=8.9 Hz,H₅), 8.24 (1,H,S,Vinyl), 7.77(1H,d,J=1.9 Hz, H₂), 7.63 (1H,dd,J=8.9, 1.9 Hz, H₆), 7.27(5H,m,Ph), 3.46(2H,q,J=7.0 Hz), 2.72 (2H,t,J=7.0 Hz), 1.96(2H, quintet, J=7.0 Hz). MS-351(M⁺, 100%), (246 (M- (CH₂)₂ Ph, 85) 217(M-NH(CH₂)₃ Ph, 20), 200(25), 186(23), 171(40), 118(80), 91(92), m/e.

### Example 2: synthesis of SSI 20

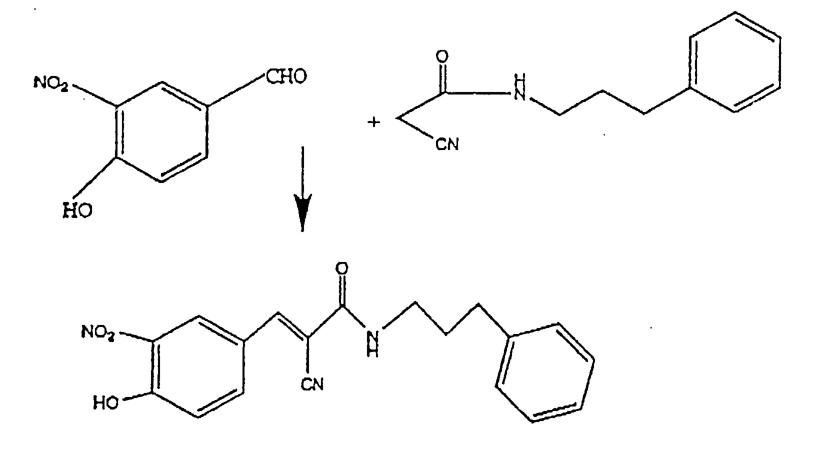

0.35g, 2 mM, 3-nitro 4-hydroxy benzaldehyde, 0.42g, 2mM, Compound 1 and 60 mg β-alanine in 15 ml ethanol were refluxed 4 hours. Cooling and filtering gave 0.48g, 65% yield, yellow solid, mp-168. NMR CDCl₃ δ 8.65(1H,d,J=2.2 Hz, H₂), 8.24(1H,S,vinyl), 8.23(1H,dd,J=8.6, 2.2 Hz, H₆), 7.26(6H,m,Ph+H₅), 3.46(2H,q,J=7.0 Hz), 2.71(2H,t,J=7.0 Hz), 1.96(2H, quintet, J-7.0 Hz). MS-351(M⁺, 75), 333(M-H₂O, 19), 246(M-(CH₂)₂ Ph, 78), 217 (M-NH(CH₂)₃ Ph, 42), 200 (M-NO₂-(CH₂)₂ Ph,69), 189(M-CONH(CH₂)₃Ph,9), 172(40), 171(40), 171(217-NO₂-, 28), 117(75), 91(100), m/e.

### Example 3: Synthesis of SSI 21

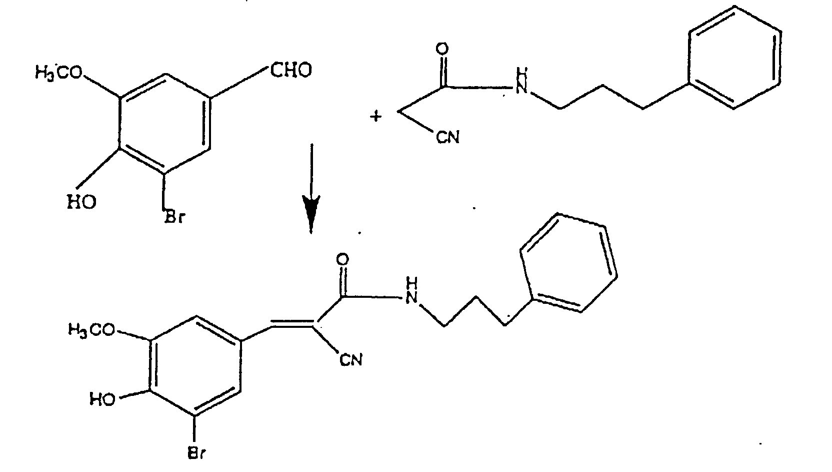

0.55g, 2.4mM, 5-bromo vanilline, 0.5g, 2.5mM, Compound 1 and 40 ml β-alanine in 15 ml ethanol were refluxed 5 hours. Cooling and filtering fave 0.71g, 71% yield, yellow solid, mp-168. NMR acetone d₆ δ 8.11(1H,S, vinyl), 7.86(1H,d,J=1.9 Hz,H₆), 7.74(1H,d,J=1.9 Hz, H₂), 7.26 (5H,m,Ph), 3.96(3H,S,OCH₃), 3.44(2H,q,J=7.0 Hz) 2.71 (2H,t,J=7.0 Hz) 1.95 (2H, quintet, J=7.0 Hz). MS-417, 415(M⁻1, 70%), 416, 414 (M+, 100%), 311, 309 (M- (CH₂)₂ Ph, 40), 297, 295 (M-(CH₂)₃ Ph, 26), 281, 279(M(CH₂)₃ Ph-O, 55), 231 (M-Br- (CH₂)₂ Ph, 84), 217, 215(50), 201(45), 200 (46), 117(30), 91(52), m/e.

### Example 4: Synthesis of SSI 22

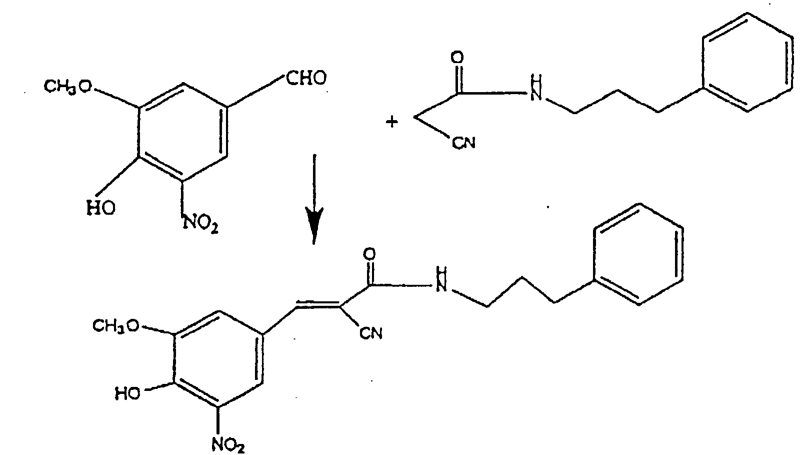

0.4g, 2 mM, 5-vitro vanilline, 0.4g, 2 mM, Compound 1 and 40 mg β-alanine in 20 ml ethanol were refluxed 4 hours. Cooling and filtering gave 310 mg, 41% yield, yellow solid, mp-106. NMR acetone d, 6 8.34(1H,d,J=1.9 Hz, H₆), 8.22 (1H,S,vinyl), 8.0 (1H,d,J-1.9 Hz, H₂), 7.25(5H,m,Ph), 4.01(3H,S,OCH₃), 3.44(2H,q,J=7.3 Hz), 2.71(2H,t,J=7.3 Hz), 1.95(2H, quintet, J=7.3 Hz). MS- 381(M+, 100%), 276(M-(CH₂)₂ Ph, 30), 268(85), 259(276-OH, 28), 245 (M-NH₂ (CH₂)₃ Ph, 43), 230(33), 223(55), 208(45), 200(30), 148(28), 117 (53), 91(82), m/e.

### Example 5: Synthesis of SSI 23

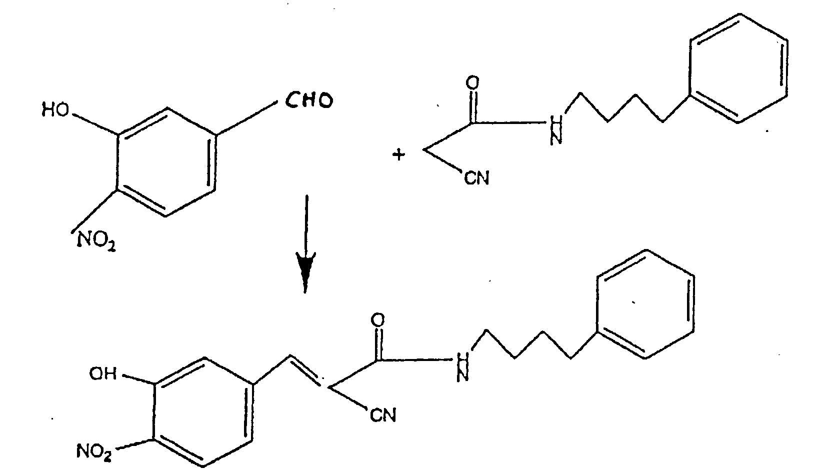

80 mg, 0.4 mM, Compound 2 60mg, 0.4 mM, 3-hydroxy 4-nitro benzaldehyde and 20 mg β-alanine in 15 ml ethanol were refluxed 4 hours. Cooling and filtering gave 74 mg, 51% yield, yellow solid, mp-148. NMR CDCl₃ δ 10.54 (1H,S,OH), 8.27(1H,S,vinyl), 8.22(1H,d,J=8.8 Hz, H₅), 7.63 (1H,d,J=1.9 Hz, H₂), 7.50(1H,dd,J=8.2, 1.9 Hz, H₆), 7.25(5H,m), 3.46(2H,q,J=7.2 Hz), 2.67(2H,t,J=7.2 Hz), 1.68(4H,m). MS-365(M⁺, 50), 274 (M-CH₂ Ph, 12), 246(M-(CH₂)₂ Ph, 7), 217(15), 171(13), 91(100), m/e.

### Example 6: Synthesis of SSI 24

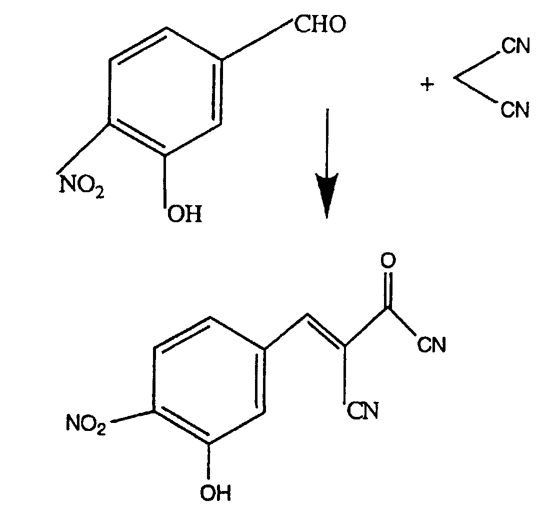

0.56g 3.3 mM, 3-hydroxy 4-nitro benzaldehyde, 0.23g, 3.5 mM, malono nitrite and 25 mg β-alanine in 15 ml ethanol were refluxed 1 hour. Cooling and filtering gave 0.62g, 86% yield, green-yellow solid, mp-176. NMR acetone d₆ δ 8.45(1H,S,vinyl), 8.30(1H,d,J=8.8 Hz, H₅), 7.79 (1H,S,D,J=2.0 HZ, H₂), 7.66(1H, dd, J=8.8,2.0 Hz, H⁶). MS-243(M⁺, 54%), 173(46%), 159(100%), 143(173-NO₁36%), 111(159-NO₂-,72%). m/e

### Example 7: Prevention of LPS toxicity and mortality in mice sensitized with galactosamine

In this model for organ failure, mice (strain (CD1)) are injected (i.p.) simultaneously with galactosamine (18 mg/mouse) and LPS (50 ng/mouse). The LD₅₀ of LPS used in this model is approximately 30,000 lower than the LD₅₀ of LPS used alone. The main toxic manifestation in this model is liver damage and mice develop severe hypoglycemia and die within 7-8 hours.

Serial blood glucose determinations were done in individual animals (2 mice per group). Administration of LPS or galactosamine alone does not affect blood glucose levels (approximately 100% mg over 24 hours) and the animals do not die. In contrast, animals injected with galactosamine and LPS develop severe hypoglycemia (as demonstrated by drops in blood glucose from approximately 100% mg to approximately 25% mg in 7-8 hours) and die within 7-8 hours. Administration of the tyrphostins SSI 3, 400 ug/mouse or SSI 17, 200 ug/mouse 2 hours prior to galactosamine and LPS, prevents hypoglycemia (blood glucose level were approximately 100% mg at 0 and the same after 24 hours) and mortality for over 5 days.

### Example 8: Effect of SSI tyrphostins on LPS-induced cytotoxicity, in vitro

Recombinant human TNFα is added to a mouse fibroblastic cell line (A9), cultured in the presence of cyclohexamine and cell viability is monitored after 20 hours. The tyrphostins tested (SSI 3 50 uM; SSI 16 2 uM; and SSI 17, 2 and 10 uM) prevent TNF toxicity to different degrees. The percentage of live cells was measured from 2 hours prior to administration until 4 hours after administration for a control and for TNF at concentrations of 0.2 ng/ml, 0.05 ng/ml, and 1.0 ng/ml. SSI 17 was most effective as judged by the doses used and by its effectiveness when added late (up to 4 hours) after TNF addition. The percentage of live cells increased sharply at first and then either remained nearly the same or slowly decreased to the lower percentage over several hours.

SSI 19 (2 and 10 uM) was also effective in preventing TNF toxicity although SSI 19 at 50 um by itself was toxic to the indicator A9 cells. SSI 23 at a high concentration (50um) was effective in preventing TNF toxicity in vitro (the percentage of live cells ranged from approximately 30% to 100% depending upon the concentration of TNF and time of administration of the tyrphostin) when added at the same time or 1 hour prior to the addition of TNF, and even when added late (2hr) after the addition of TNF.

### Example 9: Effect of tyrphostins on LPS-induced nitric oxide (NO) production by murine peritoneal macrophages

Nitric oxide was implicated in playing a role in the clinical toxicity of septic shock. Tyrphostins SSI 3, SSI 16, and SSI 17, were tested for their inhibitory activity on NO production in vitro by activated murine peritoneal macrophages (obtained 4 days following injection (i.p.) of mice with NaIO₄. Tyrphostins were added 2 hours prior to LPS and NO was determined in supernatants from cultures incubated for 24 hours. All tyrphostins tested at 50 um were found to markedly inhibit LPS induced NO production from an initial level of 40 (10 ug/ml of LPS) or 70 uM (no LPS) to approximately 20 or 30 uM.

### Example 10: Effect of tyrphostins on LPS-induced lethal toxicity

The effect of SSI 17, at different doses, on LPS induced lethal toxicity was studied. Doses of 20 ug/mouse reduced mortality by 50%, whereas a dose of 100 ug/mouse completely prevented death. In this experiment, SSI 17 was administered 2 hours prior to LPS. SSI 17 (200 ug/mouse) was almost equally effective in preventing LPS-induced mortality (approximately 80% live mice versus approximately 40% live mice with 1.5 mg/mouse of LPS alone) when administered 2 hours after LPS administration as compared to administration 2 hours prior to LPS. Several experiments were performed with 20 or 30 mice in each experimental groun).

SSI 23 (100 ug/mouse) was also found to be effective in preventing LPS-induced (2.5 and 2.2 mg/mouse) mortality (2 out of 5 mice alive after 7 days versus 0 mice alive after 1 day for 2.5 mg LAS/mouse and 4 out of 5 mice alive versus 1 alive after 7 days for 2.2 mg LPS/mouse) when administered 2 hours before or after LPS treatment. Two separate experiments were performed. The sensitivity of the mice to LPS alone (1.5 mg/mouse) differed significantly in each experiment. SSI 16, which is structurally related to SSI 17 does not prevent LPS induced toxicity in vivo at 400 ug/mouse over 7 days with 10 mice whereas SSI 17 did prevent toxicity.

Other embodiments are within the following claims.

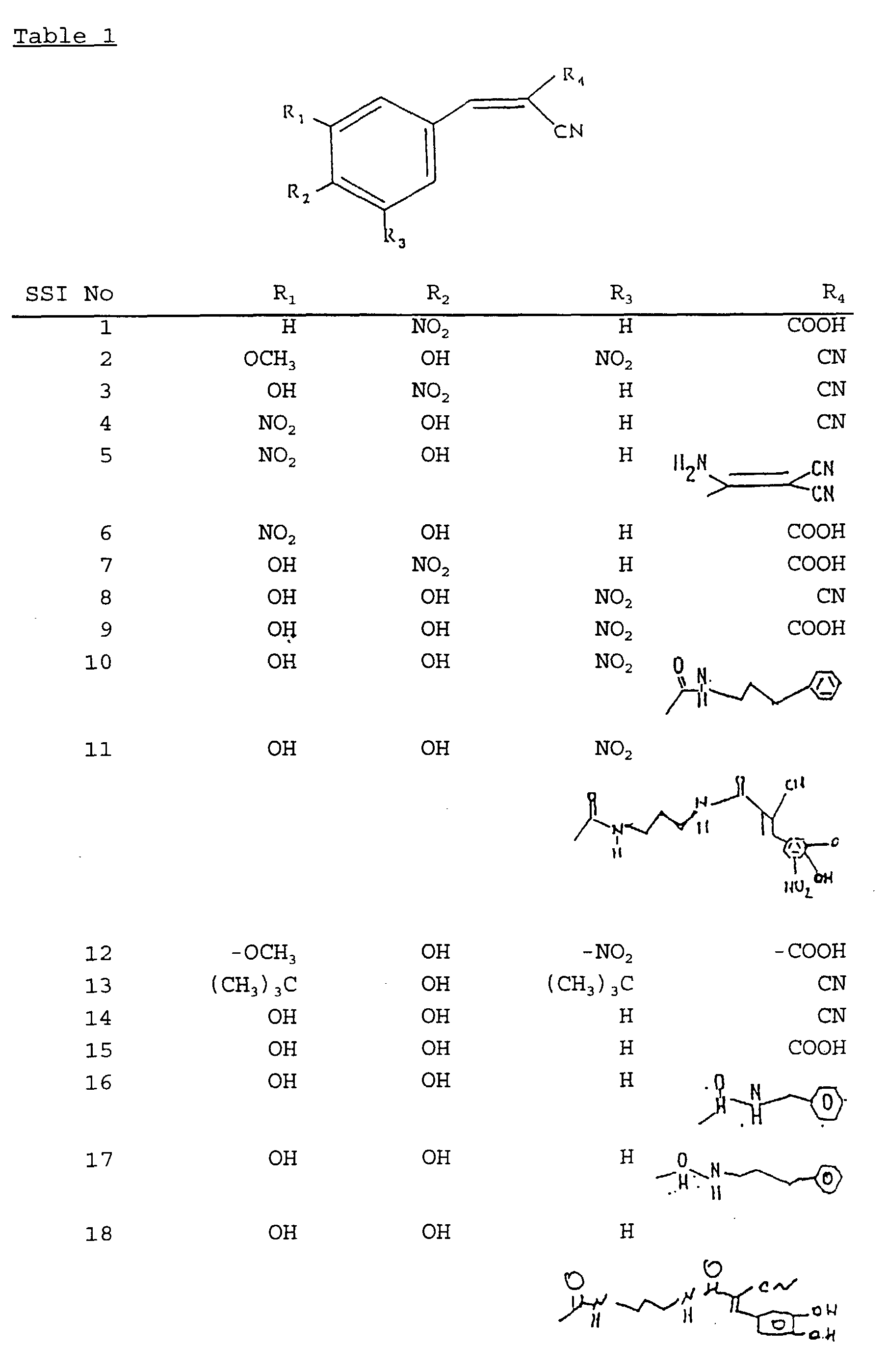

**Table 2**

| Inhibition of TNF Production in Mice After Injection of LPS by Various Tyrphostins | | | |
|---|---|---|---|
| | | ng/ml, Serum TNF-α (ng/ml Serum) | |
| | | 1hr | 2hr |
| | SSI No. | | |
| Control | 0 | 15.2 (15.1, 15.4) | |
| LPS | 0 | 51.4 (53.5, 49.4) | 29.4 (27.2, 31.9) |
| LPS | 3 | 2.8 (1.2, 4.4) | 3.6 (2.7, 4.5) |
| LPS | 9 | 17.7 (17.3, 18.2) | 11.1 (10.5, 11.7) |
| LPS | 10 | 18.7 (23.2, 19.2) | 12.9 (13.6, 12.2) |
| LPS | 11 | 16.2 (16.5, 16.0) | 8.9 (10.2, 7.6) |
| - | 3 | 16.3 | |
| - | 9 | 16.0 | |
| - | 10 | 14.0 | |
| - | 11 | 20.1 | |
| C57.BL ♂ 12 units | | | |
| AG 300 µg/mouse 2 hrs prior to LPS | | | |
| LPS 200 µg/mouse | | | |

**Table 3**

| Effect Tyrphostins on LPS-Induced NO₂- Production by Macrophages (Na104-Activated) NO₂- (µg) | | | | | | | |
|---|---|---|---|---|---|---|---|
| SSI 20 uM | 1 DAY | 2 DAY | 3 DAY | SSI 20 uM | 1 DAY | 2 DAY | 3 DAY |
| CONTROL | 44,6 | 51,2 | 68 | LPS 10 ug | 70,5 | 124 | 149 |
| 3 | 11,8 | 17,5 | 7,5 | 3 | 30,2 | 91 | 90,1 |
| 6 | 47,2 | 57 | 58,8 | 6 | 71,3 | 120,8 | 138,4 |
| 8 | 10,4 | 13,5 | 11,8 | 8 | 42,7 | 80,6 | 83,4 |
| 11 | 29 | 33,4 | 37,3 | 11 | 76,8 | 103 | 132 |
| 9 | 41,8 | 57,2 | 45,1 | 9 | 71,3 | 112,9 | 136 |
| 10 | 23,4 | 27,9 | 44,6 | 10 | 42,3 | 100,9 | 124,9 |
| | | | | | | | |

| SSI 50 uM | 1 DAY | 2 DAY | 3 DAY | SSI 50uM | 1 DAY | 2 DAY | 3 DAY |
|---|---|---|---|---|---|---|---|
| CONTROL | 44,6 | 51,2 | 68 | LPS 10 ug | 70,5 | 124 | 149 |
| 3 | 5 | 8,6 | 4,8 | 3 | 6,1 | 31,4 | 19,2 |
| 6 | 46,6 | 50,4 | 52 | 6 | 78,9 | 125 | 53,9 |
| 8 | 5,8 | 8,1 | 5,9 | 8 | 6,4 | 11,5 | 22,4 |
| 11 | 35,5 | 29,3 | 33,4 | 11 | 75,4 | 109,9 | 122,8 |
| 9 | 36 | 51 | 33 | | | | |
| 10 | 23,3 | 12,3 | 13,2 | | | | |

**Table 4**

| In Vitro Inhibition of TNF-α Production by Activated Macrophages Derived from Mouse Peritoneum by Tyrphostins | | |
|---|---|---|
| TNF-α (pg/ml) LPS | | |
| SSI No. | - | + |
| none | <5 | 46 |
| 3 | <5 | <5 |
| 8 | <5 | <5 |
| 6 | <5 | 38 |
| 9 | <5 | 17 |
| 11 | <5 | 16 |

**Table 5**

| A | | | | |
|---|---|---|---|---|
| DAY | | •mice (still alive) | Δmice | |
| 0 | | 20 | 20 | |
| 12 hr | | 19 | 20 | |
| 24 hr | | 13 | 19 | |
| 36 hr | | 12 | 18 | |
| 48 hr | | 9 | 18 | |
| 60 hr | | 7 | 18 | |
| 72 hr | | 5 | 18 | |
| 84 | | 3 | 18 | |
| 96 | | 1 | 18 | |

| B | | | | |
|---|---|---|---|---|
| DAY | •mice | ◆mice | Amice | *mice |
| 0 | 5 | 5 | 5 | 5 |
| 1 | 3 | 5 | 5 | 5 |
| 1.5 | 3 | 5 | 5 | 5 |
| 2 | 0 | 5 | 4 | 1 |
| 3 | | 5 | 2 | 1 |
| 4 | | 5 | 2 | 1 |
| 5 | | 5 | 2 | 1 |
| 6 | | 5 | 2 | 1 |

**Table 6**

| C | | | | | |
|---|---|---|---|---|---|
| DAY | •mice | | ◆mice | Δmice | *mice |
| 0 | 5 | | 5 | 5 | 5 |
| 1 | 3 | | 5 | 5 | 3 |
| 1.5 | 2 | | 5 | 4 | 3 |
| 2 | 0 | | 4 | 4 | 2 |
| 2.5 | | | 4 | 4 | 0 |
| 3 | | | 4 | 4 | |
| 4 | | | 4 | 3 | |
| 4.5 | | | 4 | 2 | |
| 5 | | | 4 | 2 | |
| 6 | | | 4 | 2 | |
| | | | | | |

| D | | | | | |
|---|---|---|---|---|---|
| DAY | •mice | ◆mice | Δmice | °mice | *mice |
| 0 | 5 | 5 | 5 | 5 | 5 |
| 1 | 3 | 4 | 5 | 4 | 5 |
| 2 | 0 | 4 | 3 | 4 | 5 |
| 3 | | 3 | 2 | 4 | 5 |
| 4 | | 3 | 2 | 4 | 5 |
| 5 | | 3 | 2 | 4 | 5 |
| 6 | | 3 | 2 | 4 | 5 |

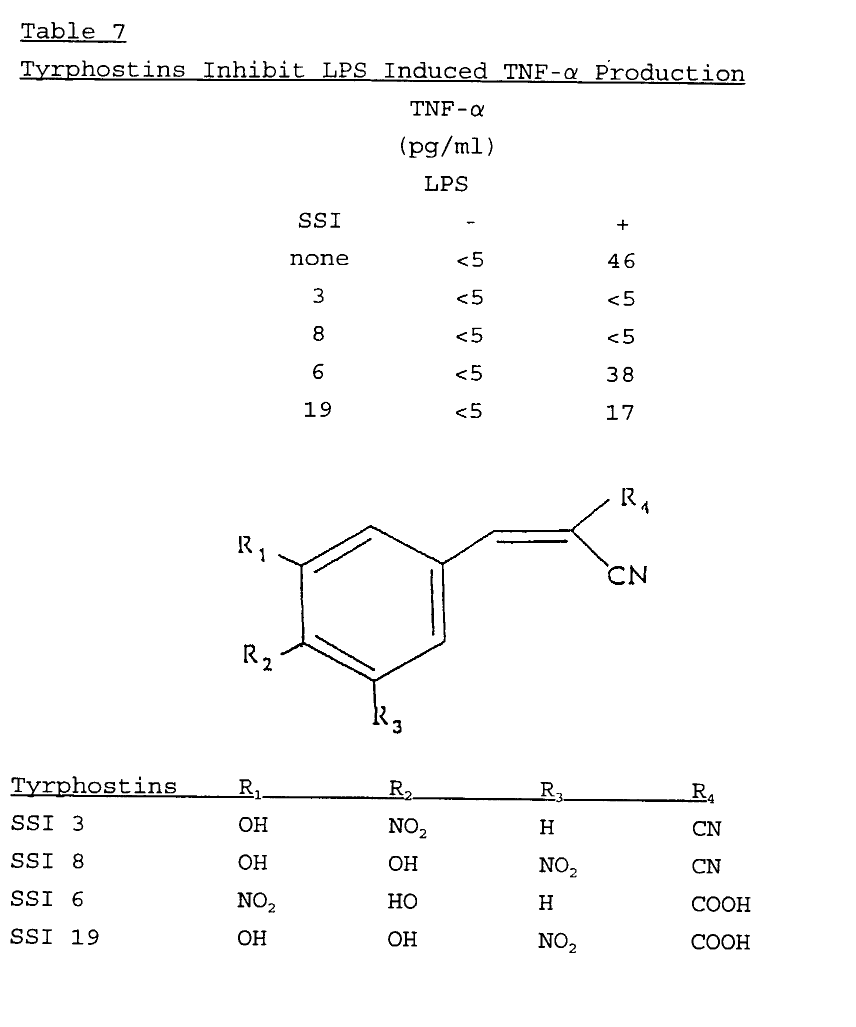

**Table 8**

| Protection of Tyrphostins Against TNF-α-Induced Cytotoxicity | | | | | |
|---|---|---|---|---|---|
| Tyrphostins | | | | | |

| TNF-α ng/ml | none | SSI 3 | SSI 8 | SSI 16 | SSI 19 |
|---|---|---|---|---|---|
| 0 | 100 | 100 | 100 | 100 | 100 |
| 0.2 | 41±2.3 | 67±3.3 | 67±4.1 | 41±4.3 | 42±3.7 |
| 0.5 | 20±1.7 | 38±2.3 | 46+2.8 | 23±2.0 | 18±2.6 |

## Claims

1. A compound of the general formula 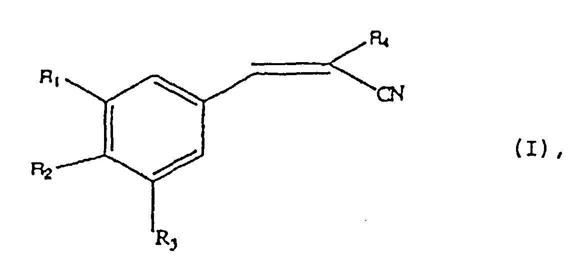 wherein in formula (I)
R₁ designates -OH, -NO₂ or C₁-C₇ alkoxy,
R₂ designates -OH or -NO₂,
R₃ designates -H, -NO₂, halogen, and -C(CH₃)₃,
R₄ designates 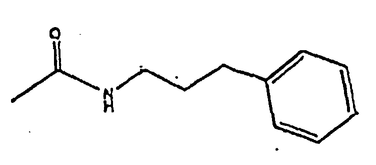 or 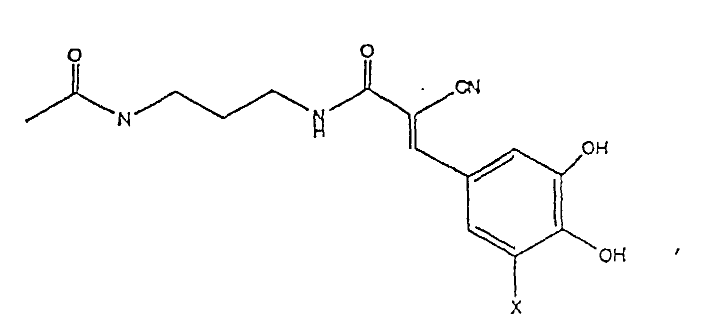 wherein X designates -H or -NO₂, and with the proviso that the compounds wherein R₁ is -OH, R₂ is -OH, R₃ is either -H or -Cl and R₄ is 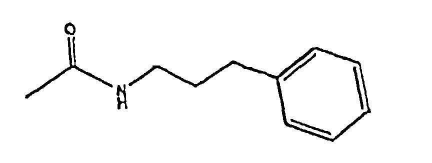 are excluded.

2. A SSI tyrphostin compound selected from the group consisting of 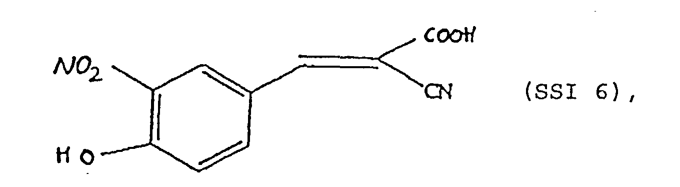 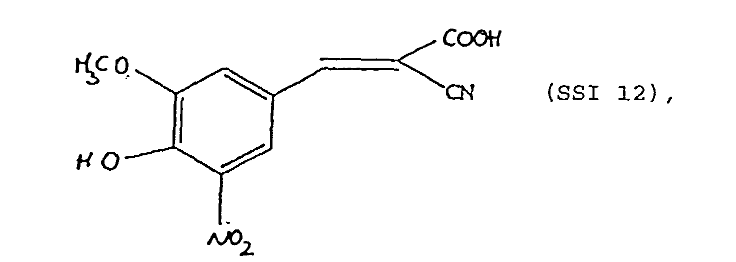 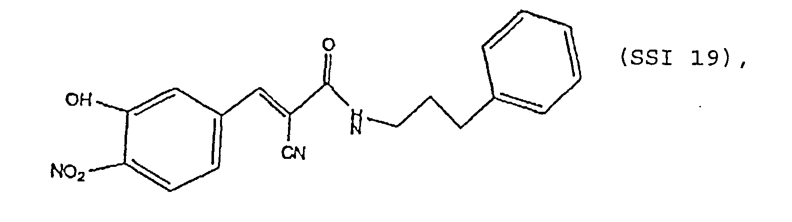 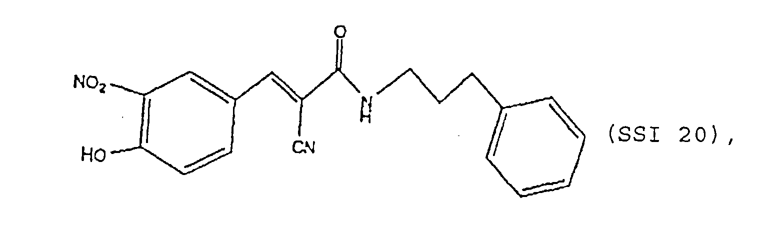 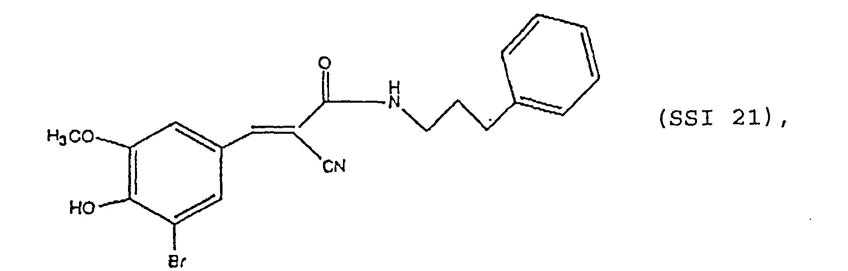 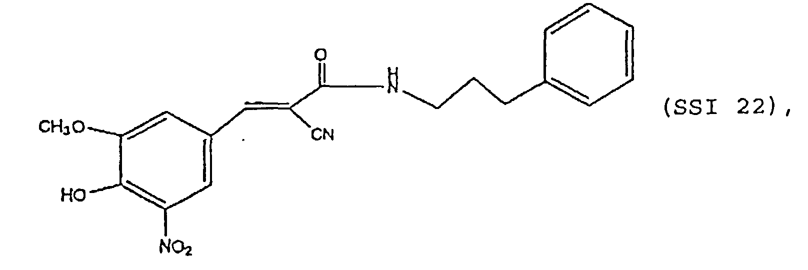 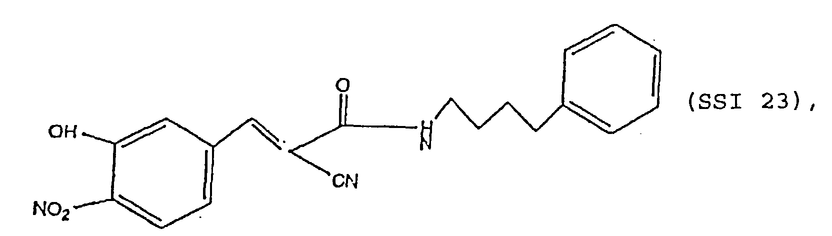 and

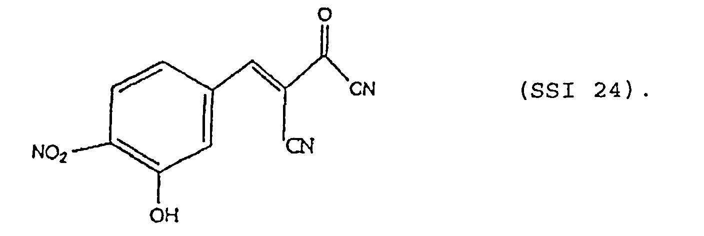

3. A method of making a SSI tyrphostin compound selected from the group consisting of SSI 19, SSI 20, SSI 21, SSI 22, SSI 23, and SSI 24 comprising the steps of exposing a benzaldehyde or substituted benzaldehyde compound to a tyrphostin or malono nitrile corresponding to a final tyrphostin of said group.

4. A pharmaceutical composition comprising: (a) a physiologically acceptable carrier or diluent; and (b) a therapeutically effective amount of a compound as defined in claim 1 or 2.

5. The pharmaceutical composition of claim 4, wherein said compound is present in a dosage from 1 mg/kg to 50 mg/kg.

6. Use of a compound selected from the group consisting of 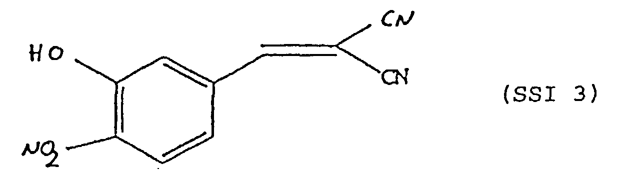 and the compounds as defined in claim 1 or 2 including the compound excluded by the proviso in claim 1, wherein R₁ is -OH, R₂ is -OH, R₃ is -Cl and R₄ is 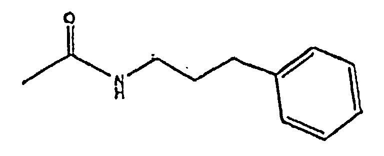 for preparing a pharmaceutical composition for treating an inflammatory disorder.

7. The use of claim 6, wherein said inflammatory disorder is selected from the group consisting of septic shock, rheumatoid arthritis, psoriasis, HIV-1, chronic granulomutotic diseases, tuberculosis, leprosy, neurological inflammatory conditions, multiple sclerosis, graft versus host disease and atherosclerosis.

8. The use of claim 6 or 7, wherein said compound is present in a dosage from 1 mg/kg to 50 mg/kg.

9. Use of a SSI tyrphostin compound of the general formula 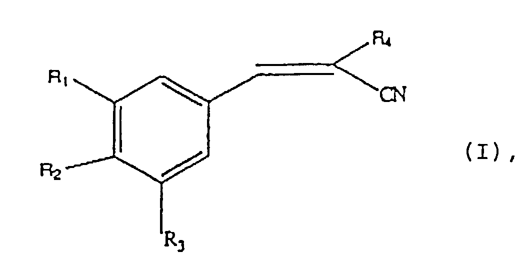 wherein in formula (I)
R₁ designates -OH, -NO₂, lower alkoxy, or -C(CH₃)₃
R₂ designates -OH or -NO₂,
R₃ designates -H, -NO₂, halogen, or -C(CH₃)₃
R₄ designates -CN, -COOH, 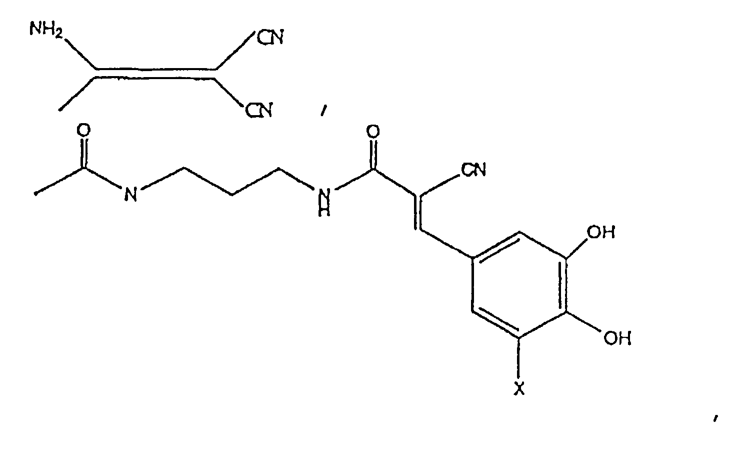 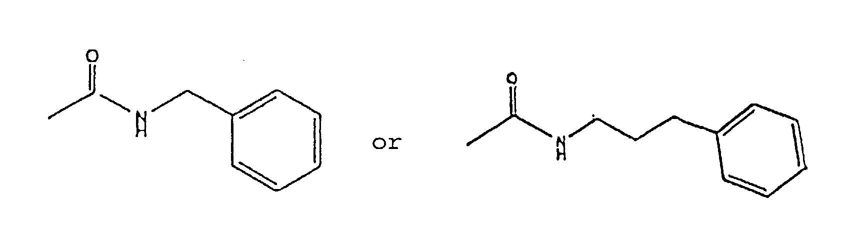 and X designates -H or nitro,
for preparing a pharmaceutical composition for preventing LPS induced toxicity, for reducing an LPS induced increase in TNF-α levels, for preventing TNF-α induced toxicity, for inhibiting production of NO₂, or for treating inflammation characterized by TNF-α related activity.

10. The use of a SSI tyrphostin for preparing a pharmaceutical composition for preventing LPS induced toxicity according to claim 9, wherein said SSI tyrphostin is selected from the group consisting of SSI 3, SSI 4, SSI 6, SSI 12, SSI 16, SSI 17, and SSI 23.

11. The use of a SSI tyrphostin for preparing a pharmaceutical composition for reducing an LPS induced increase in TNF-α levels according to claim 9, wherein said SSI tyrphostin is selected from the group consisting of SSI 2, SSI 3, SSI 6, SSI 9, SSI 10, SSI 11, SSI 12, SSI 17, and SSI 23.

12. The use of a SSI tyrphostin for preparing a pharmaceutical composition for preventing TNF-α induced toxicity according to claim 9, wherein said SSI tyrphostin is selected from the group consisting of SSI 3, SSI 16, SSI 17, SSI 18, SSI 19, and SSI 23.

13. The use of a SSI tyrphostin for preparing a pharmaceutical composition for inhibiting production of NO₂ according to claim 9, wherein said SSI tyrphostin is selected from the group consisting of SSI 3, SSI 6, SSI 8, SSI 9, SSI 10, SSI 11, SSI 16, SSI 17, and SSI 23.

14. The use of a SSI tyrphostin for preparing a pharmaceutical compositon for treating inflammation characterized by TNF-α related activity according to claim 9, wherein said inflammation is associated with a disorder selected from the group consisting of sepsis, psoriasis and AIDS related cachexia.

## Patentansprüche

1. Eine Verbindung der allgemeinen Formel 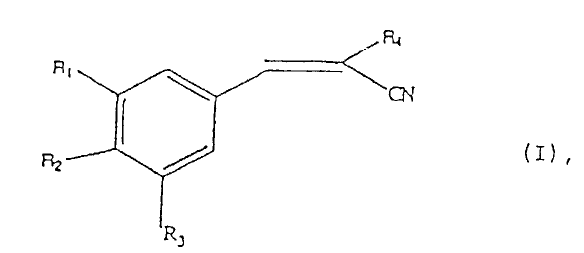 wobei in Formel (I)
R₁ -OH, -NO₂ oder C₁-C₇ Alkoxy bezeichnet,
R₂ -OH oder -NO₂ bezeichnet,
R₃ -H, -NO₂, Halogen und -C(CH₃)₃ bezeichnet,
R₄ 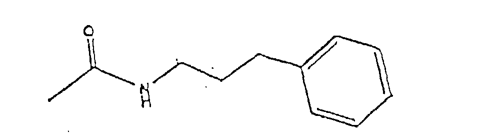 oder 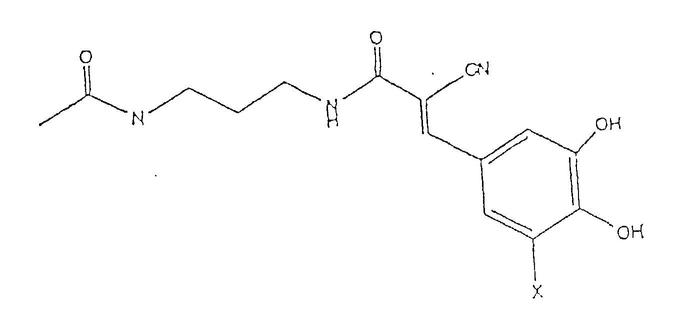 bezeichnet,
wobei X -H oder -NO₂ bezeichnet, und mit der Maßgabe, dass die Verbindungen, bei denen R₁ -OH ist, R₂ -OH ist, R₃ entweder -H oder -Cl ist, und R₄ 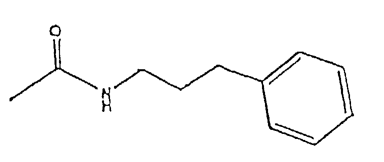 ist, ausgenommen sind.

2. Eine SSI Tyrphostinverbindung, die aus der Gruppe ausgewählt wird, die besteht aus 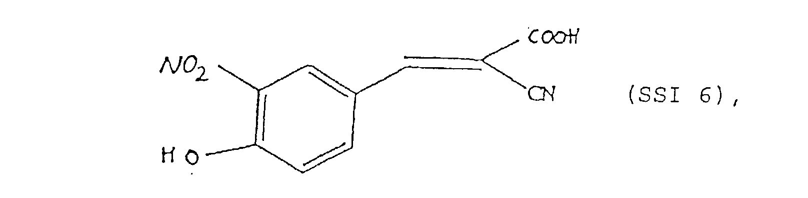 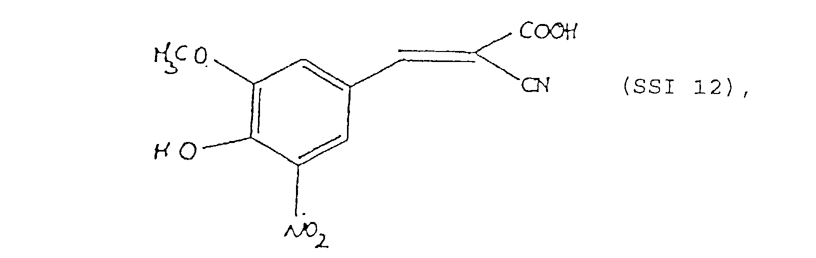 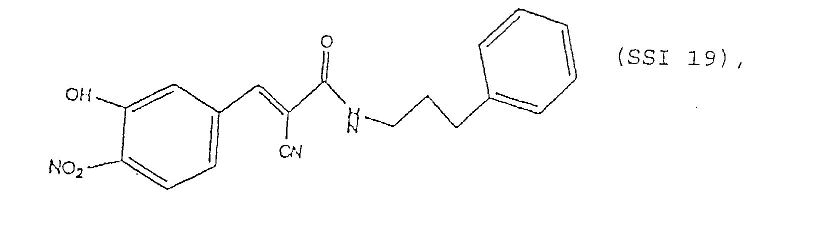 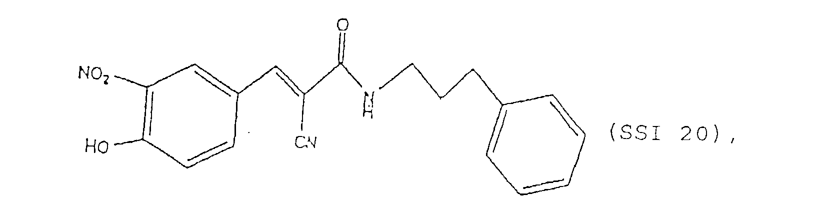 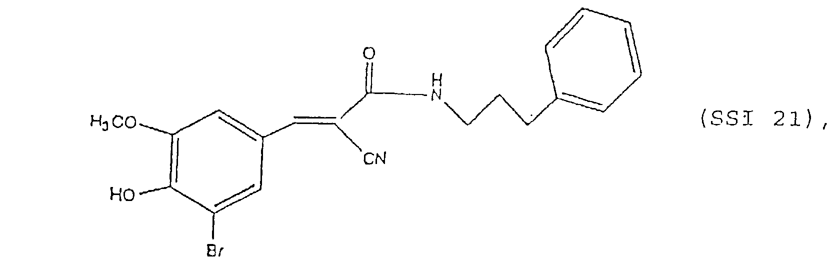 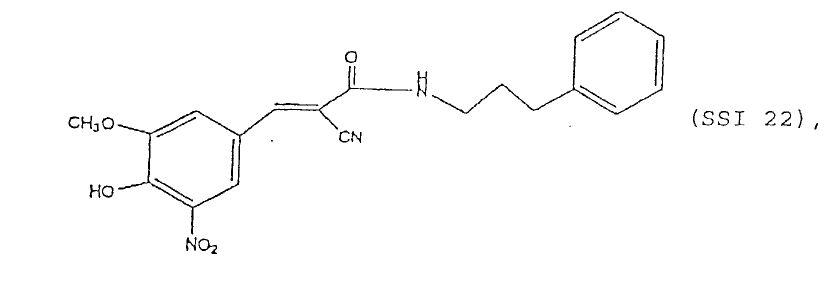 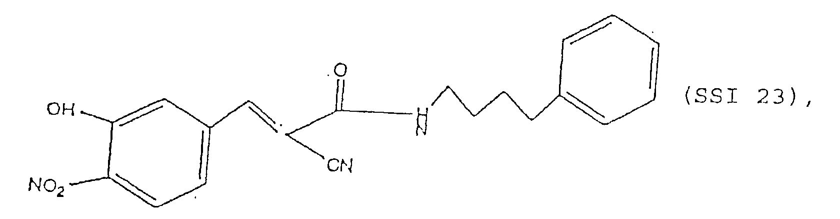 und

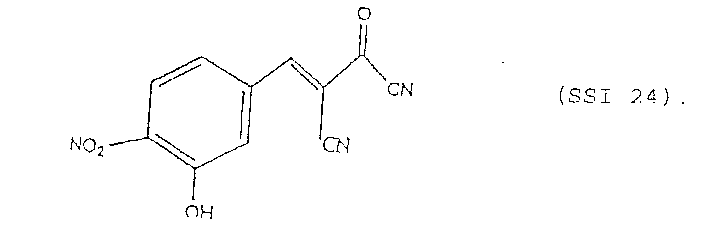

3. Ein Verfahren zur Herstellung einer SSI Tyrphostinverbindung, die aus der Gruppe ausgewählt wird, die aus SSI 19, SSI 20, SSI 21, SSI 22, SSI 23 und SSI 24 besteht, das die Schritte umfasst, eine Benzaldehyd- oder substituierte Benzaldehydverbindung einem Tyrphostin oder einem Malonsäuredinitril auszusetzen, das einem End-Tyrphostin dieser Gruppe entspricht.

4. Eine pharmazeutische Zusammensetzung, die umfasst: (a) einen physiologisch annehmbaren Träger oder ein physiologisch annehmbares Verdünnungsmittel; und (b) eine therapeutisch wirksame Menge einer in Anspruch 1 oder 2 definierten Verbindung.

5. Die pharmazeutische Zusammensetzung nach Anspruch 4, wobei die Verbindung in einer Dosierung von 1 mg/kg bis 50 mg/kg vorhanden ist.

6. Verwendung einer Verbindung, die aus der Gruppe ausgewählt wird, die aus 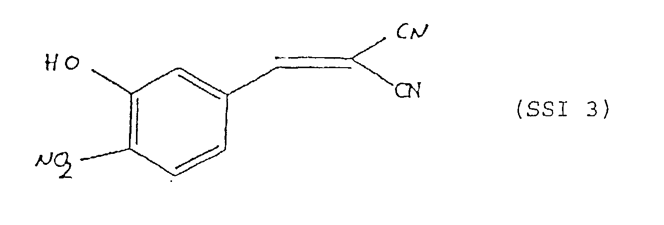 und den in Anspruch 1 oder 2 definierten Verbindungen besteht, einschließlich der Verbindung, die durch die Maßgabe in Anspruch 1 ausgenommen worden ist, bei der R₁ -OH ist, R₂ -OH ist, R₃ -Cl und R₄ 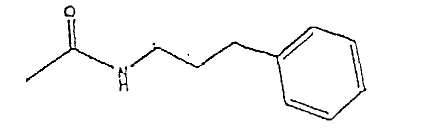 ist, zur Herstellung einer pharmazeutischen Zusammensetzung zur Behandlung einer mit Entzündung einhergehender Störung.

7. Die Verwendung nach Anspruch 6, wobei die mit Entzündung einhergehende Störung aus der Gruppe ausgewählt wird, die aus septischem Schock, rheumatoide Arthritis, Schuppenflechte, HIV-1, chronischen granulomatotische Krankheiten, Tuberkulose, Lepra, neurologischen Entzündungszuständen, Multipler Sklerose, Graft Versus Host Disease und Atherosklerose besteht.

8. Die Verwendung nach Anspruch 6 oder 7, wobei die Verbindung in einer Dosierung von 1 mg/kg bis 50 mg/kg vorhanden ist.

9. Verwendung einer SSI Tyrphostinverbindung der allgemeinen Formel 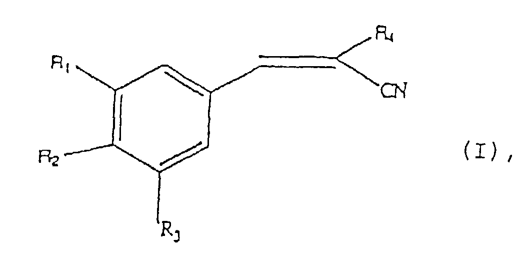 wobei in Formel (I)
R₁ -OH, -NO₂, Niederalkoxy, oder -C(CH₃)₃ bezeichnet,
R₂ -OH oder -NO₂ bezeichnet,
R₃ -H, -NO₂, Halogen, oder -C(CH₃)₃ bezeichnet,
R₄ -CN, -COOH, 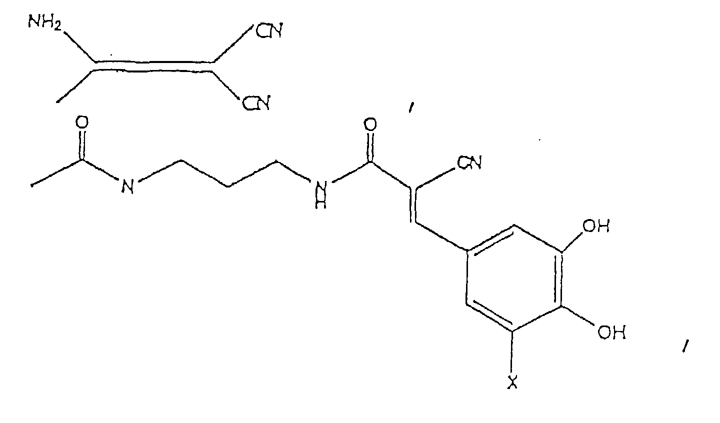 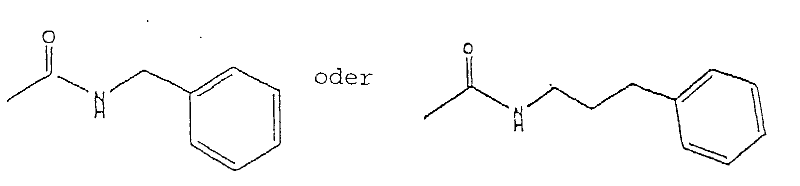 bezeichnet, und X -H oder Nitro bezeichnet, zur Herstellung einer pharmazeutischen Zusammensetzung zur Verhinderung von LPS induzierter Toxizität, zur Erniedrigung eines durch LPS induzierten Anstiegs von TNF-α Gehalten, zur Verhinderung von TNF-α induzierter Toxizität, zur Inhibierung der NO₂-Produktion oder zur Behandlung einer Entzündung, die durch eine mit TNF-α verbundene Aktivität gekennzeichnet ist.

10. Die Verwendung eines SSI Tyrphostins zur Herstellung einer pharmazeutischen Zusammensetzung zur Verhinderung von LPS induzierter Toxizität gemäß Anspruch 9, wobei das SSI Tyrphostin aus der Gruppe ausgewählt wird, die aus SSI 3, SSI 4, SSI 6, SSI 12, SSI 16, SSI 17 und SSI 23 besteht.

11. Die Verwendung eines SSI Tyrphostins zur Herstellung einer pharmazeutischen Zusammensetzung zur Reduktion eines durch LPS induzierten Anstiegs von TNF-α Gehalten gemäß Anspruch 9, wobei das SSI Tyrphostin aus der Gruppe ausgewählt wird, die aus SSI 2, SSI 3, SSI 6, SSI 9, SSI 10, SSI 11, SSI 12, SSI 17 und SSI 23 besteht.

12. Die Verwendung eines SSI Tyrphostins zur Herstellung einer pharmazeutischen Zusammensetzung zur Verhinderung von TNF-α induzierter Toxizität gemäß Anspruch 9, wobei das SSI Tyrphostin aus der Gruppe ausgewählt wird, die aus SSI 3, SSI 16, SSI 17, SSI 18, SSI 19 und SSI 23 besteht.

13. Die Verwendung eines SSI Tyrphostins zur Herstellung einer pharmazeutischen Zusammensetzung zur Inhibierung der NO₂-Produktion gemäß Anspruch 9, wobei das SSI Tyrphostin aus der Gruppe ausgewählt wird, die aus SSI 3, SSI 6, SSI 8, SSI 9, SSI 10, SSI 11, SSI 16, SSI 17, und SSI 23 besteht.

14. Die Verwendung eines SSI Tyrphostins zur Herstellung einer pharmazeutischen Zusammensetzung zur Behandlung einer Entzündung gemäß Anspruch 9, die durch mit TNF-α verbundene Aktivität gekennzeichnet ist, wobei die Entzündung mit einer Störung verbunden ist, die aus der Gruppe ausgewählt wird, die aus Sepsis, Schuppenflechte und mit AIDS einhergehender Kachexia besteht.

## Revendications

1. Un composé de formule générale : 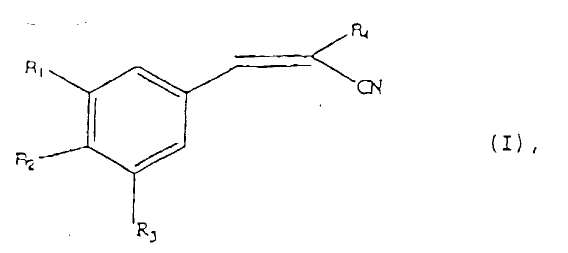 dans lequel, dans la formule (1):
R₁ représente -OH, -NO₂ ou un alcoxy en Ci à C₇ ;
R₂ représente -OH ou -NO₂;
R₃ représente -H, -NO₂, un halogène, et -C(CH₃)₃ ;
R₄ représente 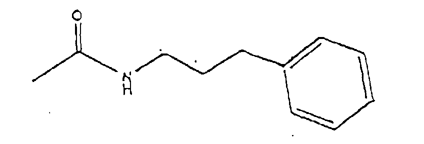 ou 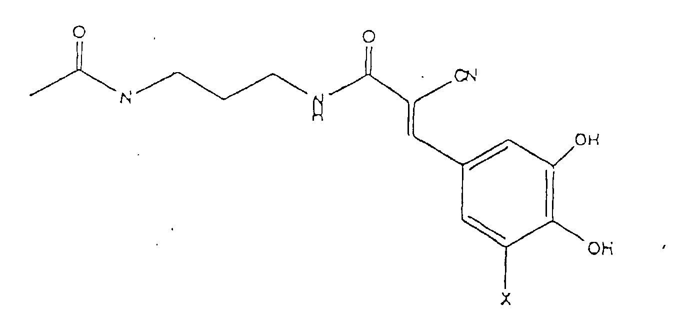 où X représente -H ou -NO₂, et avec cette condition que les composés où R₁ est un -OH, R₂ est un -OH, R3 est soit un -H, soit un -Cl, et R₄ est 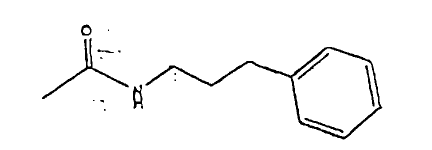 sont exclus.

2. Un dérivé de tyrphostine SSI choisi dans le groupe constitué par : 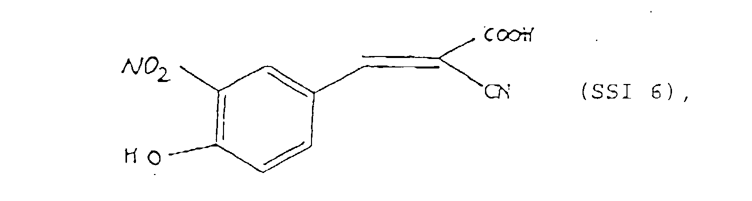 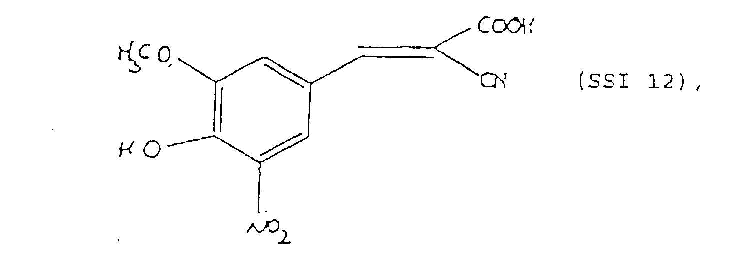 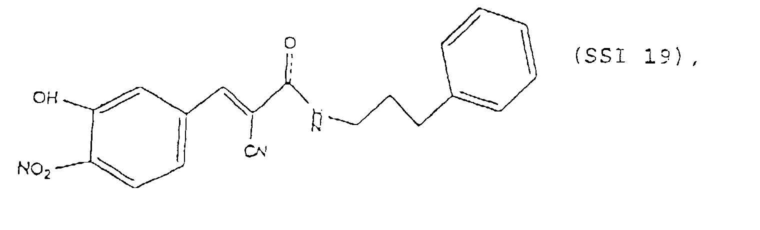 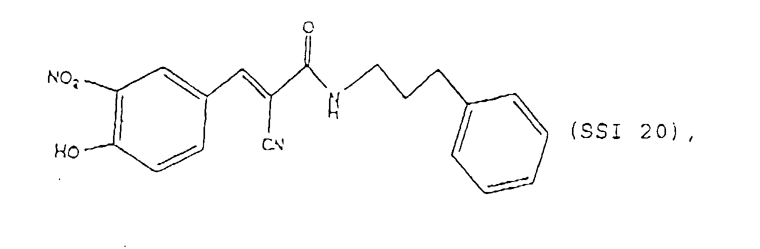 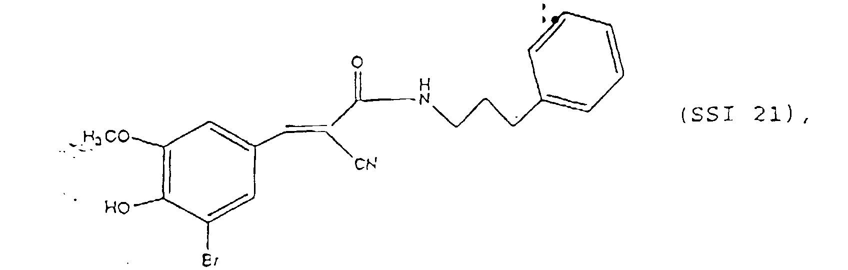 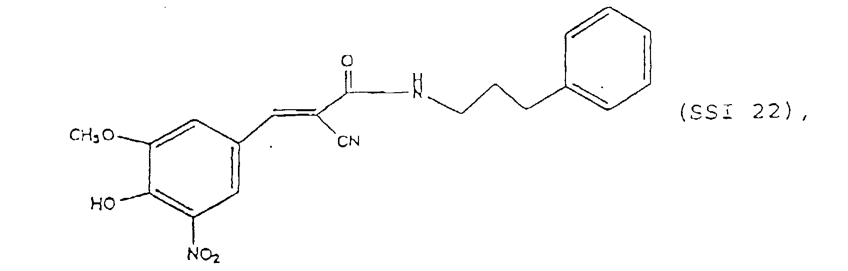 et

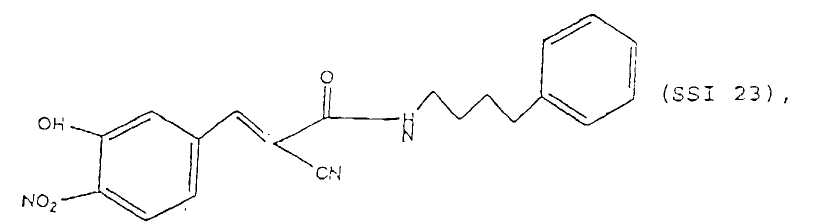

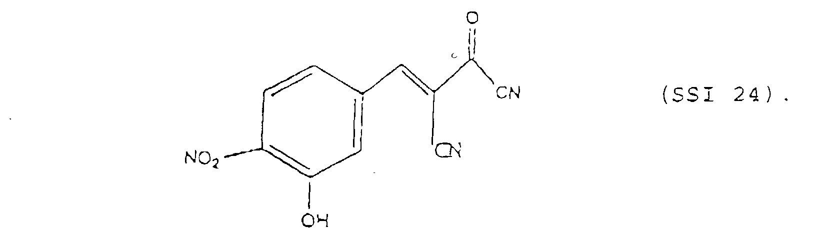

3. Procédé de préparation de dérivé de tyrphostine SSI choisi dans le groupe constitué par les SSI 19, SSI 20, SSI 21, SSI 22, SSI 23 et SSI 24, comprenant des étapes d'exposition d'un benzaldéhyde ou d'un dérivé de benzaldéhyde substitué à une tyrphostine ou un malononitrile correspondant à une tyrphostine finale dudit groupe.

4. Une composition pharmaceutique comprenant :
(a) un support ou diluant physiologiquement acceptable, et
(b) une quantité thérapeutiquement efficace d'un composé tel que défini dans la revendication 1 ou 2.

5. La composition pharmaceutique selon la revendication 4, dans laquelle ledit composé est présent à raison de 1 mg/kg à 50 mg/kg.

6. Utilisation d'un composé chimique choisi dans le groupe constitué par 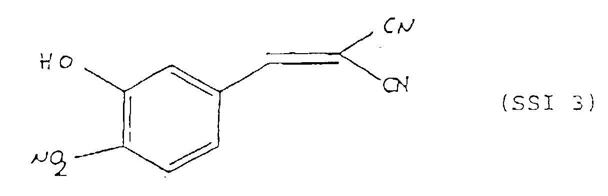 et les composés tels que définis dans les revendications 1 ou 2, y compris le dérivé exclu par la condition de la revendication 1, où R₁ représente -OH, R₂ représente-OH, R3 représente -Cl et R₄ est : 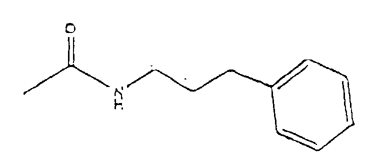 pour la préparation d'une composition pharmaceutique pour le traitement des troubles inflammatoires.

7. L'utilisation selon la revendication 6, dans laquelle ledit trouble inflammatoire est choisi dans le groupe constitué par un choc septique, une arthrite rhumatoïde, une psoriasis, le VIH-1, les maladies granulomutotiques chroniques, la tuberculose, la lèpre, un état inflammatoire neurologique, une sclérose multiple, une réaction du greffon sur l'hôte et athérosclérose.

8. L'utilisation selon la revendication 6 ou 7, dans laquelle ledit composé est présent à une dose de 1 mg/kg à 50 mg/kg.

9. L'utilisation d'un dérivé de tyrphostine SSI de formule générale : 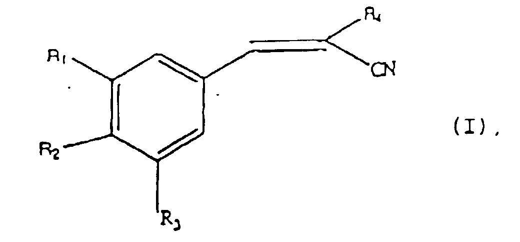 dans laquelle, dans la formule (I) :
R₁ représente -OH, -NO₂, un alcoxy inférieur ou -C(CH₃)₃;
R₂ représente -OH ou -NO₂ ;
R₃ représente -H, -NO₂, un halogène, ou -C(CH₃)₃;
R₄ représente -CN, -COOH, 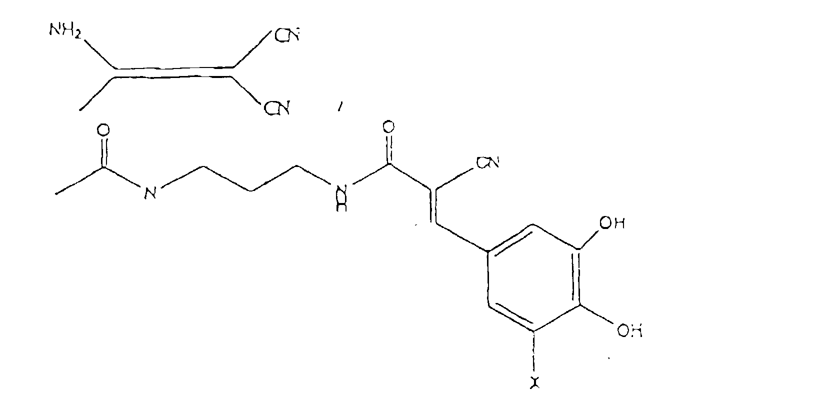 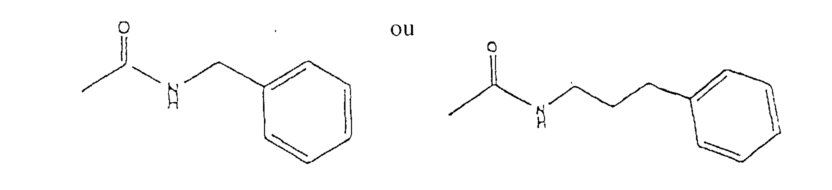 et X représente -H ou nitro,
pour la préparation d'une composition pharmaceutique pour la prévention de la toxicité induite par le LPS, pour abaisser l'élévation induite par le LPS des taux de TNF-α, pour prévenir la toxicité induite par le TNF-α, pour empêcher la production de NO₂ ou pour le traitement d'une inflammation caractérisée par une activité en relation avec la TNF-α.

10. Utilisation d'une tyrphostine SSI pour la préparation d'une composition pharmaceutique destinée à la prévention d'une toxicité induite par le TNF-ά selon la revendication 9, dans laquelle ladite tyrphostine SSI est choisie dans le groupe constitué par les SSI 3, SSI 4, SSI 6, SSI 12, SSI 16, SSI 17 et SSI 23.

11. L'utilisation d'une tyrphostine SSI pour la préparation d'une composition pharmaceutique pour abaisser l'élévation des taux de TNF-α induite par LPS selon la revendication 9, dans laquelle ladite tyrphostine SSI est choisie dans le groupe constitué par les SSI 2, SSI 3, SSI 6, SSI 9, SSI 10, SSI 11, SSI 12, SSI 17 et SSI 23.

12. L'utilisation d'une tyrphostine SSI pour la préparation d'une composition pharmaceutique pour prévenir la toxicité induite par la TNF-α selon la revendication 9, dans laquelle ladite tyrphostine SSI est choisie dans le groupe constitué par les SSI 3, SSI 16, SSI 17, SSI 18, SSI 19 et SSI 23.

13. L'utilisation d'une tyrphostine SSI pour la préparation d'une composition pharmaceutique pour inhiber la production de NO₂ selon la revendication 9, dans laquelle ladite tyrphostine SSI est choisie dans le groupe constitué par les SSI 3, SSI 6, SSI 8, SSI 9, SSI 10, SSI 11, SSI 16, SSI 17 et SSI 23.

14. L'utilisation d'une tyrphostine SSI pour la préparation d'une composition pharmaceutique pour le traitement d'une inflammation caractérisée par une activité en rapport avec la TNF-α selon la revendication 9, dans laquelle ladite inflammation est associée à un trouble choisi dans le groupe constitué par les sepsis, psoriasis et cachexie en relation avec le SIDA.
